# EUROPEAN PATENT SPECIFICATION

(11) **EP 3 618 609 B1**
(45) Date of publication and mention of the grant of the patent: **02.08.2023**
(21) Application number: 18794492.1
(22) Date of filing: 03.05.2018
(51) Int. Cl.: A01H 1/02, A01B 79/00, A01M 21/04

(54) **PLANT TREATMENT SYSTEMS AND METHODS**
PFLANZENBEHANDLUNGSSYSTEME UND -VERFAHREN
SYSTÈMES ET PROCÉDÉS DE TRAITEMENT DE PLANTES

(30) Priority: 04.05.2017 US 201762501393 P
(43) Date of publication of application: 11.03.2020
(73) Proprietor: Arugga A.I Farming Ltd, 4050000 Even Yehuda (IL)
(72) Inventor: GELTNER, Iddo, 4050000 Even Yehuda (IL)
(74) Representative: Lavoix
(86) International application number: PCT/IL2018/050487
(87) International publication number: WO 2018/203337

(56) References cited:
- WO-A2-2015/013723
- WO-A2-2015/013723
- GB-A- 2 133 664
- GB-A- 2 133 664
- JP-A- 2011 200 196
- JP-A- 2011 200 196
- JP-A- 2012 120 472
- JP-A- 2017 012 137
- US-A1- 2016 353 661
- US-A1- 2016 353 661

## Description

### TECHNOLOGICAL FIELD

The present invention relates to automated treatment of plants in industrial agriculture, such as in green-house grown plants. Specifically, treatment of plants include, for example, targeted mechanical pollination, localized prevention and/or treatment of disease, and inhibition/control of degree of pollination and/or plant growth.

### BACKGROUND

Crops need a lot of care, starting from maintaining plants health all over the plant life cycle and followed by pollinating the flowers and insuring healthy crops. With the continuous increase in population and the increased interest in healthy life while decreasing expenses, the traditional ways of agriculture are challenged every day. As known, the majority of crops we eat or use are mainly pollinated by wind or insects. However, for various reasons these natural processes do not exist or do not function optimally. For example, decrease in quantity or disappearance of insects from the farming area, or environmental conditions which limit the ability of insects to move around and pollinate. In greenhouses, for example, wind and insects cannot enter and pollinate. In addition, the growing need for food and cost reduction requires higher efficiency, which subsequently drives farmers to improve fruit set and yield beyond natural pollination.

Depending on conditions and cost, solutions range from manual pollination in various methods, to artificial introduction of insects such as honey-bee hives to farming areas, or industrially grown insects (e.g. the bombus bee). In addition, there exist mechanical solutions such as vibrating cables holding the plants, or manually vibrating individual plants, trusses or flowers in plants which can self-pollinate.

In many areas, manual pollination is prohibitive due to availability of labor and cost. Bees have several disadvantages too: they require certain environmental conditions, are sensitive to pesticides, can escape (from greenhouses) or pollinate more lucrative crops nearby. They can also transfer viruses and fungi.

US patent application 20160353661 describes a method of pollinating a plant which includes receiving, with a processing circuit, plant data regarding a plant having flowers, and controlling, by the processing circuit, operation of a robotic device to selectively pollinate a portion of the plurality of flowers based on the plant data. The robotic device includes sensors configured to acquire plant data, a pollination device configured to pollinate flowers of a plant, a collection device configured to collect pollen, and a pollination prevention device configured to prevent a flower from being pollinated.

US20180065749 describes methods and systems of pollinating crops, the systems include one or more unmanned vehicles including a pollen applicator configured to collect pollen from a flower of a first crop and to apply the pollen collected from the flower of the first crop onto a flower of a second crop and a sensor configured to detect presence of the pollen applied to the flower of the second crop by the pollen applicator to verify that the pollen collected from the flower of the first crop by the pollen applicator was successfully applied by the pollen applicator onto the flower of the second crop.

JP2011200196 discloses a pollination robot provided with a traveling device, an identification apparatus to identify a normal flower and an unopened flower, a vibrator to vibrate the flower and perform pollination treatment, a pollinated position memorizing device to memorize positions finished with pollination treatment, a controller to control the vibrator in a manner to vibrate only the flower identified to be a normal flower by the identification apparatus and pollinate flowers excluding flowers at positions identified to be finished with pollination treatment by the pollinated position memorizing device, and a harvesting device to harvest fruits.

### GENERAL DESCRIPTION

The present invention provides novel techniques for treating plants during all of the growth and fruitage cycles including, but not limited to, monitoring plant conditions such as plant health, readiness for pollination and post pollination , and intervening in each of the aforementioned conditions or stages by applying the suitable treatment in order to insure a maximum effectivity and efficiency with respect to yield.

The systems and methods of the present invention are autonomous and based on robotic treatment device(s) that can approach the specific plant or portion of the plant to monitor and treat every plant in a farming area autonomously and without human intervention.

Further, the highly effective systems and methods of the invention are resource, energy and cost efficient in that the treatment applied is targeted and local, down to the sub-plant level and down to a single flower or a specific portion of the flower.

In some aspects, the present invention provides a novel technique for plant treatment by pollinating flowers, specifically automatically pollinating flowers, e.g. for use in industrial agriculture. The system induces vibrations in one or more regions of a plant in order to selectively pollinate one or more flowers on the plant. The one or more regions, referred to occasionally as a portion of the plant, can be for example a plant stem, branch, leaf stalk, leaf, a group of flowers, a flower, or a portion of a flower. The system can detect the flower targets, determine whether they are ready for pollination and determine whether they were not pollinated yet, in order to efficiently and quickly pollinate as much flowers as required in optimal time and energy, and by this cover large amounts of plants.

In another aspect, the present invention provides systems and methods for plant treatment that include inhibiting over pollination of flowers in order to optimize yield.

In yet another aspect, the present invention provides systems and methods for plant treatment that includes local identification of disease and selectively treating disease.

Autonomous systems and methods, in accordance with the invention, have several important advantages , *inter alia:* the techniques are not limited by the availability of human labor; not sensitive to temperature and other conditions required for efficient use of bees; not sensitive to pesticides which may kill the bees or require their removal for a certain amount of time; not limited to areas where bees can be used; it does not pose any threat to employees, e.g. stinging by bees; not confined by the fact that bees cannot perform selective pollination in order to prevent over pollination requiring subsequent pruning; and not affected by the fact that bees can also damage flowers if visiting them too many times.

Thus according the present invention, there is provided a plant treatment system comprising the features of claim 1.

In some embodiments, said at least one plant treatment device comprises a vibrating element being configured and operable to contact said at least portion of the plant while vibrating to thereby induce said vibrations thereto.

In some embodiments, said at least one plant treatment device may be configured and operable as a plant pollination device, such that said induced vibrations are configured to cause pollination of at least one flower within said at least portion of the plant. The at least one plant treatment device may comprise a filter configured and operable to block microbes, viruses and/or other harmful objects and prevent delivering them to the at least portion of the plant with the air or fluid flow.

In some embodiments, said plant treatment apparatus further comprises an additional plant treatment device comprising a substance delivery device configured and operable to locally deliver or spray one or more treatment substances onto one or more regions of said at least portion of the plant, said treatment substances comprising one or more of the following: a medicament for treating plant disease, a plant hormone inducing plant growth, a pesticide that kills pests, or a plant damaging substance that prevents growth and/or pollination. The substance delivery device may be associated with said one or more treatment channels. The plant treatment device and the additional plant treatment device may be associated with said at least one fluid flow channel. The substance delivery device may be configured and operable to spray pollen towards at least one flower within said at least portion of the plant.

In some embodiments, said at least one plant treatment device comprises a vibrating element, wherein said control system is configured and operable to provide a predetermined profile of the vibrations of the vibrating element by controlling at least one of frequency, amplitude and duration of the vibration.

In some embodiments, said at least one treatment device is configured and operable to induce the vibrations by generating an air flow, wherein said control system is configured and operable to provide the predetermined profile of the air flow by controlling at least one of the following parameters: number of train pulses of air, time gap between train pulses, number of pulses in each train pulse, time gap between two pulses in each train pulse, amplitude of pressure in each pulse, duration of each pulse.

In some embodiments, the optical sensor and the fluid flow treatment channel are configured with a predetermined fixed relative orientation between axis of line of sight of the optical sensor and axis of propagation of the directional fluid stream. The predetermined fixed relative orientation may comprise an offset and/or angular difference between the axis of the line of sight of the optical sensor and the axis of propagation of the directional fluid stream. The at least portion of the plant being treated may be located within a field of view of the optical sensor. A light collecting plane of said optical sensor may be located adjacently to a fluid exit aperture of said directional fluid stream. The optical sensor and the fluid exit aperture may be fixedly attached.

In some embodiments, said plant treatment apparatus further comprises a pollen transport device configured and operable to collect pollen from a container on vehicle or in farming area and deliver the collected pollen to a pistil of at least one flower within said at least portion of the plant. The pollen transport device may have a patterned surface configured to adhere the pollen being collected to said surface.

In some embodiments, said sensing system further comprises one or more environmental sensors configured and operable to provide the sensing signals indicative of one or more environmental conditions in a vicinity of said at least portion of the plant. The plant treatment apparatus may further comprise an additional plant treatment device comprising an environment conditioning device being configured and operable to modify at least one of temperature and humidity of a surrounding of said at least portion of the plant. The control system may be configured and operable to operate said environment conditioning device. The environment conditioning device may be associated with said one or more treatment channels. The plant treatment device, the substance delivery device and the environment conditioning device may be associated with said at least one fluid flow channel.

In some embodiments, the control system is configured and operable to process the sensing signals and, upon determining that a flower within said at least portion of the plant is to be pollinated, generate corresponding operational data for said at least one plant treatment device to induce said vibrations in the at least portion of the plant.

In some embodiments, wherein the sensing system comprises one or more environmental sensors configured and operable to provide the sensing signals indicative of one or more environmental conditions in a vicinity of said at least portion of the plant, the sensing signals may be indicative of unfavorable conditions for pollination, and the control system may generate operational data for said substance delivery system to deliver or spray a hormone that induces parthenocarpic fruit growth. The sensing signals may be indicative of a disease of said at least portion of the plant or pest in a surrounding of or on said at least portion of the plant, and the control system may generate operational data for said substance delivery system to deliver or spray a medicament or a pesticide respectively.

In some embodiments, the plant treatment system further comprises a sterilization and/or cleaning and/or disinfecting assembly configured and operable to sterilize and/or clean and/or disinfect said at least one plant treatment device. The sterilization and/or cleaning and/or disinfecting assembly may comprise at least one of the following: a hot air blower, a cleaning material applicator and a cleaning or disinfecting or sterilizing material sprayer.

In some embodiments, the plant treatment apparatus comprises a navigation and tracking assembly configured and operable to bring the plant treatment apparatus to a vicinity of said at least portion of the plant to thereby enable treating said at least portion of the plant by the plant treatment system. The navigation and tracking assembly may comprise a robotic arm carrying said plant treatment assembly, and the control system may be configured and operable to controllably move the robotic arm in three dimensions. The navigation and tracking assembly may comprise a ground vehicle configured and operable to controllably transport the plant treatment apparatus to the vicinity of said at least portion of the plant. The navigation and tracking assembly may comprise at least one of the following: one or more optical sensors, and a positioning sensor. The navigation and tracking assembly may comprise an inertial moment unit configured and operable to determine spatial movement path of the robotic arm to thereby optimize plant treatment process time and energy.

In some embodiments, the control system is configured and operable to determine, based on said sensing signals, whether at least one flower on said portion of the plant is ready for pollination, by comparing said sensing signals with reference data comprising images of flowers ready for pollination, and/or by processing said image data to identify presence of a flower in the image(s) and identify readiness of the flower(s) for pollination by identifying flower parameters indicative of existence or absence of pollination, and/or by utilizing trained artificial intelligence.

In some embodiments, the control system is configured and operable to analyze the sensing signals from at least the optical sensor and determine a condition of said at least portion of the plant while being treated and after the treatment, and generate corresponding feedback data, enabling decision making about modification of at least one parameter of the treatment affecting the vibrations induced in the at least portion of the plant.

In some embodiments, the plant treatment apparatus further comprises a pollination inhibiting device configured and operable to prevent pollination to occur to one or more flowers and/or prevent growth and blossoming of additional flowers within said at least portion of the plant, while minimizing damage to nearby parts of the plant. The pollination inhibiting device may comprise a laser device configured and operable to irradiate said at least portion of the plant with predetermined laser parameters to thereby damage said at least portion of the plant.

In some embodiments, the at least one treatment device is configured and operable as a pollination inhibiting device configured and operable to generate said fluid stream with a predetermined high temperature, while maintaining the fluid stream directionality by controlling size of fluid stream exit, to burn one or more regions of said at least portion of the plant and prevent pollination to occur to one or more flowers and/or prevent growth and blossoming of additional flowers within said at least portion of the plant, while minimizing damage to nearby parts of the plant.

According to another aspect of the invention, there is provided a plant treatment apparatus according to claim 20.

According to another broad aspect of the invention, there is provided a method for plant treatment comprising the features of claim 25.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** illustrates by way of a block diagram an exemplary embodiment of a plant treatment system according to the invention;
**Fig. 2** illustrates a non-limiting example of a treatment apparatus which induces vibration in at least part of a plant by way of air flow;
**Fig. 3** illustrates a non-limiting example of a treatment apparatus which induces vibration in at least part of a plant by way of contact;
**Fig. 4a****-c** illustrates 3 non-limiting examples of a treatment apparatus which delivers directed localized substance or fluid to at least part of the plant;
**Fig. 5** illustrates a device to control the aperture of the air flow or fluid delivery treatment channel;
**Fig. 6** illustrates a non-limiting example of a treatment system to deliver pollen to the flower by way of contact;
**Fig. 7a****-c** illustrate 3 non-limiting examples of a treatment apparatus which cause pollination inhibition to a part of the plant;
**Fig. 8** illustrates a non-limiting example of mounting of the treatment device together with imaging sensors;
**Fig. 9** illustrates a non-limiting example of mounting of the treatment apparatus which induces vibration in at least part of a plant by way of contact, together with a cleaning/sterilizing/disinfecting system;
**Fig. 10** illustrates a non-limiting example of a navigation and tracking assembly for use with several different treatment devices ;
**Fig. 11** illustrates a non-limiting example of mounting several treatment apparatuses that induce vibration by way of air flow on a robotic vehicle;
**Fig. 12** illustrates a non-limiting example of a pattern of the air flow to induce vibration in at least a portion of a plant; and
**Fig. 13** illustrates a non-limiting example of a pollination inhibition apparatus mounted on a robotic vehicle.

### DETAILED DESCRIPTION

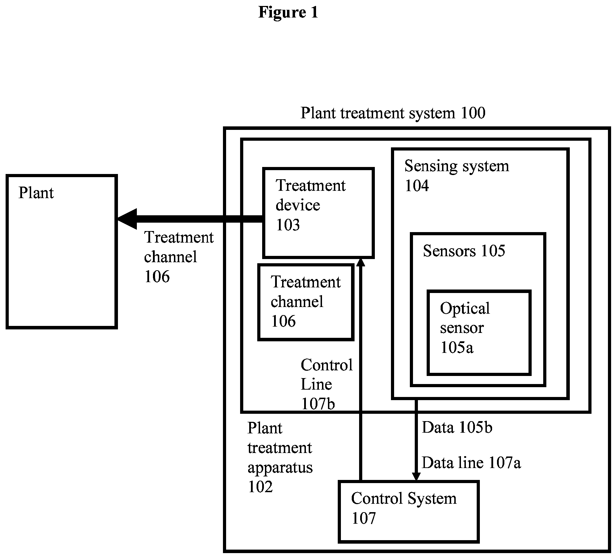

Reference is made to Fig. 1 that depicts a general schematic diagram of the features of a non-limiting embodiment of a plant treatment system 100 for treating at least a portion of a plant, in accordance with the invention. The plant treatment system 100, includes a plant treatment apparatus 102 and a control system 107 connected to and communicating with the plant treatment apparatus 102, e.g. via data and/or control lines 107a and 107b. The plant treatment apparatus 102 includes one or more treatment channels 106 and at least one plant treatment device 103 associated with the one or more treatment channels. The plant treatment apparatus 102 also includes a sensing system 104 that includes one or more sensors 105 configured and operable to provide, to the control system 107, sensing signals 105b indicative of a condition of the at least portion of the plant.

Accordingly, the plant treatment system 100 is configured and operable to monitor the plant during all of its growth stages, including the stage of flower blossoming, pollination and fruitage, by the sensing system 104 and once a predetermined condition of the plant, relating to one or more of the plant growth stages, is identified, the plant treatment system 100 is configured and operable to apply a corresponding treatment by the at least one plant treatment device 103 associated with one or more treatment channels 104. For example, the plant treatment system 100 identifies, by suitable sensor(s) of the sensing system 104, a condition related to pollination, e.g. whether one or more flowers on the plant are ready to be pollinated and as a result, the plant treatment system 100 operates the at least one plant treatment device 103 in order to pollinate the one or more flowers. In some embodiments, and as will be further detailed below, the plant treatment system 100 can monitor the health of the plant, by suitable sensor(s) of the sensing system 104, and upon identifying that the plant suffers from a specific disease, the plant treatment system operates the at least one treatment device 103 to apply a corresponding treatment to the diseased plant, such as by delivering a suitable drug or medicament.

As described, the sensing system 104 monitors, by its one or more sensors 105, a condition of the plant, generates a corresponding sensing data and sends the sensing data to the control system 107. The control system 107 receives the sensing data, via the data line 107a, and processes the sensing signals to determine the condition of at least portion of the plant, then operates the at least one plant treatment device 103, by generating operational data and sending it via the control line 107b, to apply a corresponding treatment to the at least portion of the plant.

It should be noted that while, in this specific example, the illustration shows that the control system 107 is a separate element, it can be configured in other ways too. For example, the control system 107 can be an integral part of either the sensing system 104 or the at least one treatment device 103 or can be distributed there between. In that case, the data and control lines are merged into one transmission/communication line. Further, it is noted that the control system 107 may be located inside or outside the plant treatment apparatus 102. For example, the control system 107 can run on an external server that communicates with the other system's elements via network, whether wired or wireless network.

The at least one treatment device 103 is configured and operable to apply treatment to the at least one portion of the plant by controllably inducing a vibration pattern in the at least one portion of the plant. The vibration pattern is costumed to the desired kind of treatment to be applied, by controlling the parameters of the profile of the vibration pattern. The at least one treatment device 103 can be configured to apply the vibration pattern to one or more regions in the at least one portion of the plant to thereby achieve the required treatment in the minimum energy and/or time. The vibration pattern, induced in the one or more regions of the at least one portion of the plant, can be applied by the plant treatment device 103 in a contact or contactless manner, as will be further described below.

As mentioned above, the plant treatment apparatus 102 includes one or more treatment channels 106 which the at least one treatment device is associated with one or more of them. The one or more treatment channels include channel(s) that the plant treatment device 103 uses/requires in order to apply the treatment to the plant. The treatment channel(s) 106 can form an internal part of the plant treatment system or in some cases can be external to it. The treatment channel(s) 106 can be an entry, an intermediate or an exit part with respect to the one or more plant treatment devices 103. It should be noted that, in some embodiments, more than one plant treatment device can be associated with a single, common, treatment channel. In some embodiments, a single plant treatment device can be associated with more than one treatment channel. For example, the treatment channels can include a fluid flow channel configured and operable to provide a flow of fluid, either in gas or liquid or aerosol phase, that the plant treatment device utilizes to apply the treatment. In one specific example, the fluid flow channel is utilized by the plant treatment device in order to generate an air-flow and to blow air towards one or more regions of the at least portion of the plant.

The one or more sensors 105 of the sensing system 104, capable of sensing signals indicative of a condition of the portion of the plant under examination, include at least an optical sensor 105a configured and operable to provide the sensing signals indicative of image data of the at least portion of the plant. The optical sensor 105a can be configured as any optical sensor known in the art. Specifically, the optical sensor 105a can be a camera pointing directly towards the portion of the plant under examination, or can have or be associated with an aperture pointing towards the portion of the plant (e.g. by utilizing an optical fiber while the sensor itself has no direct line of sight with the portion of the plant), or can have a field of view that includes the portion of the plant, etc. The image data can be indicative of a variety of conditions of the portion of the plant that their identification invites a respective treatment by a suitable treatment device. For example, the image data can teach about diseases of the plant, readiness of one or more flowers to pollination, existence of already pollinated flowers, etc.

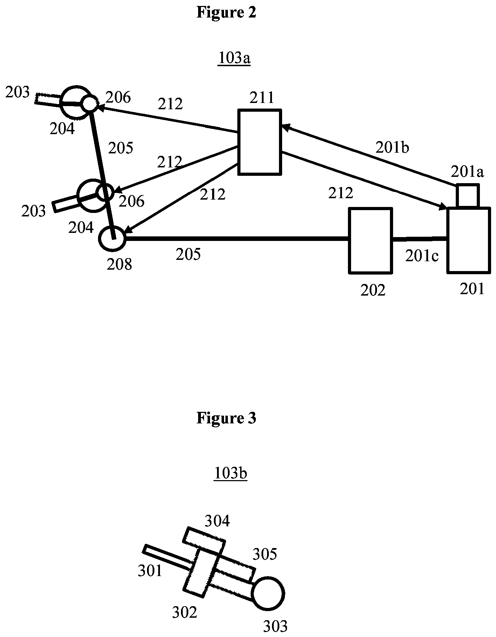

Fig. 2 depicts a non-limiting example of a plant treatment device 103a which induces vibration of a predetermined profile in at least part of a plant, according to the invention. In this example, the treatment device induces the vibration pattern in the at least portion of the plant contactlessly by providing a controlled and directional air flow towards the at least portion of the plant. The directional air flow exiting the distal side of the plant treatment device 103a and propagating towards the target portion of the plant, e.g. a single flower, is actually a defined air/fluid stream that has predetermined velocity profile, temporal profile (e.g. being configured as pulses with gaps in between) and spatial profile including direction and volume that can be defined for example by an axis of propagation and stream width. Therefore, the treatment device of Fig. 2 is associated with a treatment channel that includes a fluid flow channel. In the described example, the treatment device includes a controller 211 which controls the components of the treatment device through control lines 212. The controller 211 can be part of the control system or can be a direct part of the treatment device 103a as described above. In some embodiments, the controller is configured for data communication with the control system for receiving therefrom operational data indicative of the sensing data provided by the sensing system. A compressor 201 compresses air to a predetermined value or to a value determined by controller 211 which receives the pressure reading from pressure gauge 201a through data line 201b and operates compressor using control line 212. The compressor 201 compresses the air which fills a tank 202 through tube 201c. The tank 202 is connected to fluid flow applicators 203 through tubing 205. Compressed air is delivered to fluid flow applicators 203 upon demand by controlling valves 206 by controller 211 through control lines 212. Air can be delivered to fluid flow applicators 203 independently of each other by controlling each valve 206 separately. Air pressure is further controlled by pressure regulator 208 which is controlled by controller 211 though control line 212.

Air compressor 201 can compress air to the maximum value that the device is planned to use, and by controlling regulator 208 and valves 206, each fluid flow applicator 203 can receive an independently set pressure. The air compressor 201 can be equipped with a microbial filter or other filter (not shown) in order to prevent delivering microbes/viruses or other harmful objects through applicator to plants or portions of plants.

Tubes 205 can be flexible, in order to allow movement of applicators 203 in order to direct air delivery onto required part of plant, by mounting applicators 203 on movable mounts 204, also controlled by controller 211 through control lines 212.

Fluid flow applicators 203 can provide a predetermined profile of air flow adjusted to provide the required treatment to the at least portion of the plant. The predetermined profile of air flow can be a sequence of air pulses by intermittently opening and closing valves 206 at required timings, controlled by controller 211, and by changing air pressure by regulator 208.

Fig. 3 depicts another non-limiting example of a plant treatment device 103b which induces vibration of a predetermined profile in at least part of a plant, by way of direct physical contact with the at least portion of the plant. Accordingly, the treatment device 103b is associated with a treatment channel comprising contact force application. The treatment device 103bincludes a contact applicator 301 attached to a vibrating element 302, both connected to a mount 303. The mount 303 can connect the treatment device to a suitable part of the treatment apparatus 102 as will be described below.

The contact applicator 301's length may vary depending on the plant treated. The length may be changed manually by extending or retracting the contact applicator on its holding element attaching it to the vibrating element 302. It can also be controlled by a motor 304 which can vary its length upon commands from a controller 305 that can be configured similarly to controller 211 as described above. The contact applicator 301's rigidity/stiffness can vary depending on target plant. The contact applicator 301 may be rigid if target plant portion to be vibrated is thick/stiff/large/hard to vibrate, or more flexible if portion of plant to be vibrated is small or gentle.

Vibrating element 302 can be constantly vibrating, or can be operated by controller 305 for example upon signal from proximity sensor or force gauge (not shown) placed on the holding element of applicator. Vibration amplitude and frequency may also be changed by controlling vibrating element through controller 305.

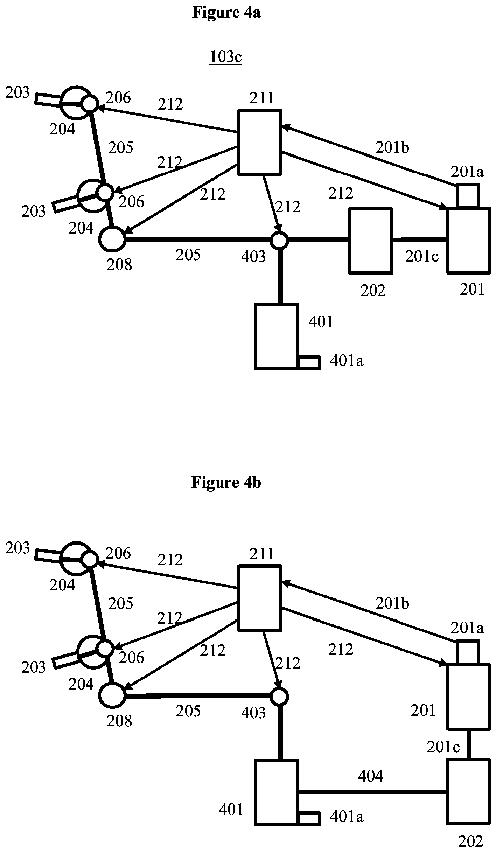

Figs. 4a-c depict yet another non-limiting example of a plant treatment device 103c for applying a treatment to at least part of a plant, according to the invention. Specifically, the plant treatment device 103c is configured and operable for directed and localized fluid and/or substance delivery to at least a portion of a plant. Accordingly, the treatment device 103c is associated with a treatment channel for dispensing material, and in some embodiments, the treatment channel is a fluid flow channel. The treatment device 103c is similar in some of its features to the treatment device 103a described in Fig. 2. The treatment device 103c includes all the elements of the treatment device of Fig. 2 with an additional fluid reservoir 401. The reservoir 401 is connected to tubes 205 through valve 403, which is controlled by the controller 211 through a control line 212. The reservoir 401 can be filled through connector 401a.

In order to deliver fluids to a portion of the plant, valve 403 is operated such that it allows both pressurized air to flow from tank 202 and fluid from reservoir 401. Together, the mixture of air and fluid are delivered through tubes 205 to fluid flow applicators/apertures 203 by opening valves 206. In some embodiments, reservoir 401 can contain a powder, which is delivered to a portion of the plant in the same manner as the fluid.

Another non-limiting embodiment of the fluid delivery treatment device 103c is described in Fig. 4b. Here, pressurized air from tank 202 is used to pressurize fluid in reservoir 401 through tube 404. The pressure is controlled by compressor 201 which is operated by controller 211. The pressurized fluid in reservoir 401 is delivered to plant through valve 403 which is controlled by controller 211 through control line 212. Reservoir 401 can also be pressurized by connecting pressurized air through connector 401a.

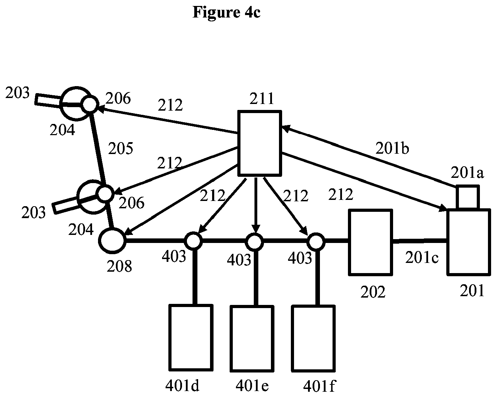

Both non-limiting embodiments described in Figs. 4a and 4b can be expanded to include more than one reservoir. An example of such embodiment is described in Fig. 4c with 3 reservoirs 401d-f. This embodiment enables the treatment device to deliver for example 3 types of fluid to the plant, either separately or as mixtures, by controlling timing of valves 403 by controller 211 through control lines 212. Similarly, one reservoir 401f may contain a cleaning fluid which will flush the tubing 205, valves 403 and 206, regulator 208 and fluid applicators 203 between the application of the different fluids/materials.

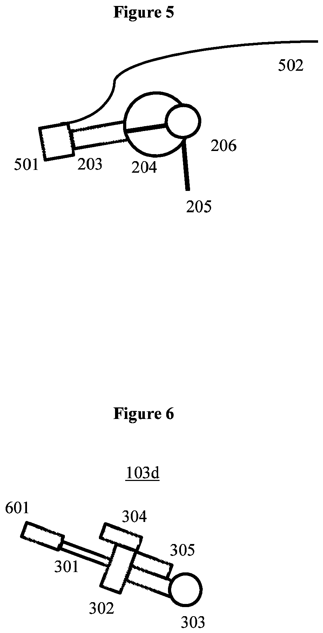

Fig. 5 depicts a non-limiting example of an applicator/aperture 203, shown in Figs. 2 and 4, with an adjustable opening 501 at its distal tip. The figure shows the aperture 203, on its mount 204, with tubing 205 connected through valve 206. Adjustable opening 501 is connected to controller 211 through control line 502. When used with system for fluid delivery as described in Figs. 4a-c, the adjustable opening size can be changed in accordance with the type of fluid. For example, when fluid is more viscous the opening 501 can be enlarged. Opening's size can be changed in order to adjust application of fluid between directed spraying to aerosol. When used with air flow device described in Fig. 2, opening's size can change in order to affect air flow pattern from directed, when opening's size is large to divergent, when opening's size is small. The size can be changed depending on type of portion of plant to be vibrated, its distance from the applicator 203, or in order to change flow rate in order to control amplitude of vibration. When vibration is induced on a large portion of a plant, or when the distance is large or when amplitude of vibration is large, the opening size needs to be enlarged to increase air flow. When the portion of the plant to be vibrated is close or small, the induced vibration should be gentle and the opening size can be decreased. In general, opening's size should be kept to a minimum in order to minimize air flow and conserve energy, especially when dealing with a treatment device operated on batteries, or when pressurized air is delivered from a reservoir of the treatment device.

Fig. 6 describes a non-limiting example of a plant treatment device 103d for delivery of pollen to at least one flower on a plant. The treatment device 103d is similar to the treatment device shown in Fig. 3, with a brush or pad 601 placed at the distal tip of the applicator 301. The brush or pad is brought into contact with the flower on the plant. Pollen preloaded on brush/pad 601, e.g. by immersing the brush/pad in a pollen reservoir, is delivered to a female organ of flower by vibrating brush against organ by operating vibrating element 302. Vibration initiation, duration, frequency and amplitude are controlled by controller 305.

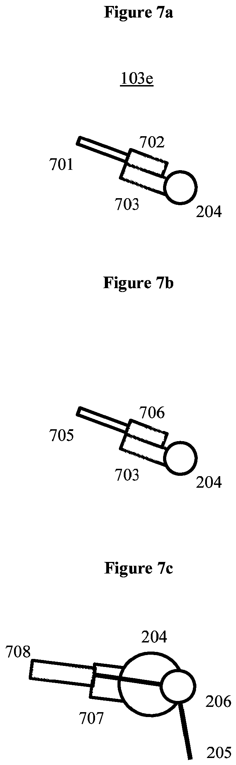

Figs. 7a-7c describe non-limiting examples of a pollination- inhibition plant treatment device 103e that can be used to damage a portion of the plant. In one embodiment, shown in Fig. 7a, the treatment device includes a laser 702, and an applicator 701, through which laser beam is directed towards the portion of the plant, positioned on a holder 703 connected to a mount 204 which can point the beam to the required direction. The laser beam can be pulsed, having long pulses or short picosecond or femtosecond pulses, and can have various wavelengths, e.g. in the IR, visible, or UV spectrum.

In another embodiment, shown in Fig. 7b, hot directed air stream is blown towards part of the plant, created by blower 706 and directed towards plant by fluid flow applicator/aperture 705. Size of Opening of applicator/aperture 705 should not be too small (on the order of 1-3mm) in order for hot air to be directional and by this minimize damage to surrounding portions of the plant.

In yet another embodiment, shown in Fig. 7c, the distal tip of the plant treatment device shown in Fig. 2 is configured with heater 707 and applicator/aperture 708. Air is fed through tubes 205 and valve 206 and heated with heater 707 before being directed towards plant through applicator/aperture 708. All components are positioned on mount 204.

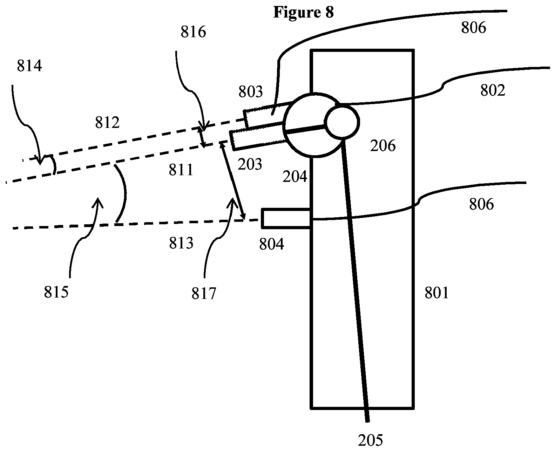

Fig. 8 depicts a non-limiting example of a plant treatment apparatus with a plant treatment device and a sensing system according to the invention. Proximal tips of plant treatment devices described in Figs. 2, 4 and 7 are placed on mount 204. This mount can be positioned on a fixed post 801 as depicted in Fig. 8. The mount 204 can have two angular degrees of freedom in order to point applicator/aperture 203 towards portion of plant to be treated. An imaging device 803, forming one optical sensor of the sensing system 104, can be placed on mount 204 adjacent to aperture 203. Imaging device 803 is positioned relative to aperture with fixed offset 816. This offset can be translational only, i.e. pointing to same direction but shifted, or can have an angular offset 814 as well. If line of sight 812 of imaging device 803 is parallel to pointing direction 811 of aperture 203, the offset is only translational. Another fixed imaging device 804, forming another optical sensor of the sensing system, can be positioned on post 801. This imaging device 804 is offset relative to aperture 203 with offset 817, and angle 815 is the angle between pointing direction 811 of aperture 203 and line of sight 813 of imaging device 804. Angle 815 depends on position of mount 204. Either or both of the imaging devices can be used to point aperture 203 towards portion of plant to be treated. Both imaging devices can send image data through data lines 806 to controller 211. The controller 211 can determine position and distance of portion of plant to be treated and in turn point aperture 203 to target portion of the plant by controlling mount 204 position through control line 802. The positioning of mount 204 can be determined using one imaging device, or both. If offset of aperture and imaging device is fixed, as in the offset of aperture 203 and imaging device 803, and this offset is known to controller 211, then pointing of aperture 203 can be done by pointing imaging device 803 with an offset relative to portion of plant to be targeted. If offset is adjustable, as in the case of aperture 203 and imaging device 804, the distance to target can be measured by image analysis of image data provided by imaging device 804, and with known translational offset 817, controller 211 can point aperture correctly to the targeted portion of the plant. While this requires the additional data of distance, the advantage is that an imaging device located at a large offset from the aperture may see targets not visible to imaging device located adjacent to aperture. Two or more imaging devices can overcome the issue of hidden targets, or at least increase chance of not missing targets. In addition, image data from two or more imaging devices placed with an offset from each other can be analyzed stereoscopically to find the distance to the target.

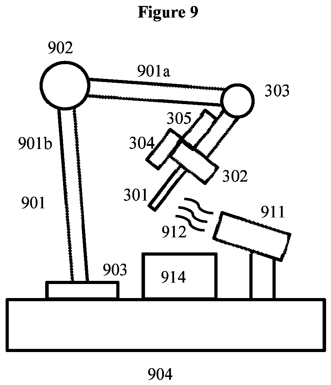

Fig. 9 depicts an example of mounting the treatment device 103b described in Fig. 3. As described above, the treatment device 103b is configured and operable to induce vibration on a portion of a plant through contact. The treatment device 103b includes the contact applicator 301, its vibrating element 302, motor 304, controller 305 and mount 303. The mount connects the treatment device to a manipulator arm 901, which in this specific example includes two arm sections 901a and 901b, connected with joint 902 and having mount 303 acting as another joint too. The arm 901 is placed on a base 903 which can also act as another joint.

The arm's length and degrees of freedom, determined by the number and lengths of arm sections, joints and their respective degrees of freedom, should be such to enable reaching by contact all required portions of the plant which are planned to be treated, including but not limited to highest and lowest parts of the plant. In addition, the overall reach of the manipulator arm can be such as to allow reaching portions of plant from different angles of approach, for example reaching a leaf from underneath, or reaching a stem from one or more sides. The purpose is both to be able to contact portions of plant at different angles of approach and prevent damage to other plant portions when approaching, or to allow imaging portions of plant from various angles when configuring distal tip with one or more imaging devices.

Also shown in Fig. 9 is a cleaning device 911 intended to clean/disinfect/sterilize applicator 301 or any other part of a treatment device that contacts the plant, such as brush/pad 601 described in Fig. 6. Cleaning device 911 can be configured as a hot air blower blowing hot air 912, or as a dispenser/sprayer to deliver any other material 912 required whether liquid, aerosol or spray, on applicator 301. Cleaning device 911 can be placed on same base 904 together with the manipulator arm 901 so that the arm can place applicator 301 in a fixed position known to be reached by the cleaning material applied by cleaning device 911. Another option is placing a tank 914 with cleaning/disinfecting/sterilizing material in a fixed position on base 904, such that the manipulator arm 901 can dip applicator 301, or any other plant-contacting part of the plant treatment device(s), inside the tank 914 in order to clean/disinfect/sterilize it. The timing of cleaning can be controlled by user or automatically by the control system, according to completion of actions taken by the plant treatment device/apparatus or for example by time elapsed, or by environmental conditions, or by disease and/or pests known to be in the farming area or detected by the sensing system during operation.

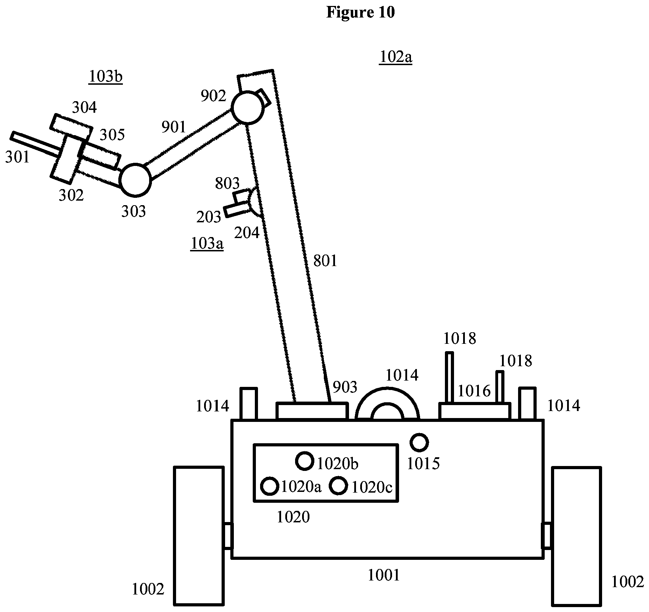

Fig. 10 depicts a non-limiting example of a plant treatment apparatus 102a configured according to the invention. The plant treatment apparatus includes a plurality of plant treatment devices and a sensing system. In this example, one plant treatment device 103b for inducing vibration through contact and one plant treatment device 103a for inducing vibration through air flow are shown and both are mounted on same post 801. Post 801 is placed on base 903, which is described in Fig. 9 and can be static or movable. It should be noted that any combination of the treatment devices described in previous figures can be placed together on post 801. The sensing system includes an optical sensor (imaging device 803) and a set of environmental sensors 1020: temperature sensor 1020a, humidity sensor 1020b and light/ambience sensor1020c. These sensors can detect environmental conditions in the area surrounding the plant to be treated.

As also shown in the figure, the plant treatment apparatus 102a includes a navigation and tracking assembly 1000 configured and operable to bring the plant treatment apparatus to a vicinity of the at least portion of the plant to thereby enable treating the at least portion of the plant by the plant treatment system. The navigation and tracking assembly includes a movable platform 1001, e.g. a ground vehicle, that can carry the treatment apparatus adjacent to plants to be treated. Vehicle 1001 can be a robotic vehicle, with wheels 1002, operated by motors within vehicle 1001 body. Robotic vehicle can approach plants autonomously using navigation and tracking sensors. For example, robotic vehicle can be equipped with imaging sensors 1014 at front and sides, radar (either MW based or laser based) 1015, and other peripheral sensors as required.

Movement of the robotic vehicle 1001 can be controlled by a dedicated processing unit 1016 and/or by the control system 107. Processing unit 1016 can include wireless communication, an inertial moment unit and GPS, with their respective antennas 1018. The processing unit 1016 collects data from cameras, sensors, inertial moment unit and GPS to guide vehicle 1001 along plants in farmed area. The processing unit controls the motors that operate the wheels 1002 as well as the treatment devices. Wireless communication may be used to communicate with other vehicles to coordinate coverage of farming area or with a central computer. Processing unit 1016 can replace controller 211, or vice versa, to control the treatment apparatus components, namely motors, mounts, valves, imaging sensors, manipulator arms, compressors, and regulators, all described in previous figures, and partially shown in Fig. 10.

While some of the components mentioned are not displayed in Fig. 10 for the sake of clarity, all elements described in previous figures can be placed on vehicle 1001 to support the treatment device(s).

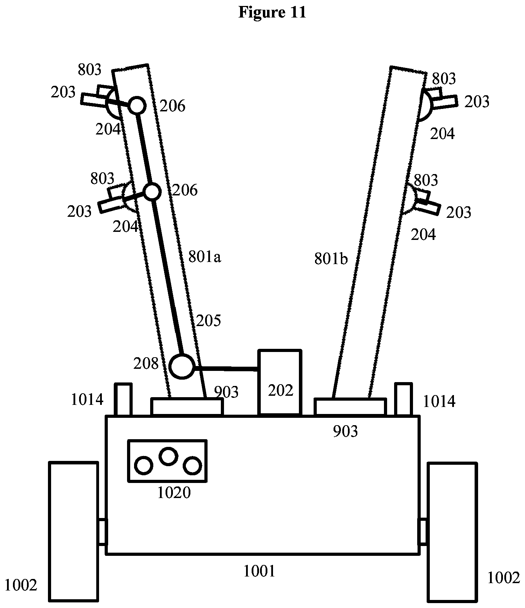

Fig. 11 displays another possible non-limiting setting of a treatment apparatus carried by vehicle 1001. Two posts 801a and 801b that carry one or more treatment devices and optionally sensors of the sensing system, are placed on both sides of the vehicle in order to treat plants on both sides simultaneously or alternately (without the need to turn the vehicle). As shown, different treatment devices can be placed on the same post to enable, for example, treatment of two or more portions of the plant being at different heights simultaneously. If a manipulator arm is placed on a post (not shown), this serves to shorten the required arm extension, thereby increasing the distal tip placement accuracy and reducing the motor strength, size, and cost needed to turn the arm joints. It should be noted that a contact-less vibration inducing treatment device typically moves with 2 degrees of freedom, and contact-based treatment device typically requires at least 3 degrees of freedom (practically may require more to both position applicator in contact with portion of plant, the need to avoid other parts of plant as well as the need to place device at a specific angle relative to portion of plant and the fact that portion of plant which may be a stem which for example can have a random direction/position), which complicates the system.

On each carrying vehicle more than one holding post can be placed in order to treat several plants in parallel, for example one post on each side (to treat plants on both side of row) and/or more than one post one every side of the vehicle to treat two or more consecutive plants simultaneously.

As appreciate, the plant treatment apparatus/system can be a mobile system that can move in a farming area and carry the treatment devices/apparatus adjacent to every plant. The transport/navigation system can be based on wheels, as shown in Fig. 10, or on rails or tracks placed along plant rows in the farming area. The rails can have marked places which can signal the system to stop at each plant. The rails can be placed on the ground or in the air. Together with the control system, location devices such as GPS, and peripheral cameras, the plant treatment apparatus/system can detect plants, register their location and track their treatment in order to return to same portions/flowers or avoid their treatment if they have been treated already.

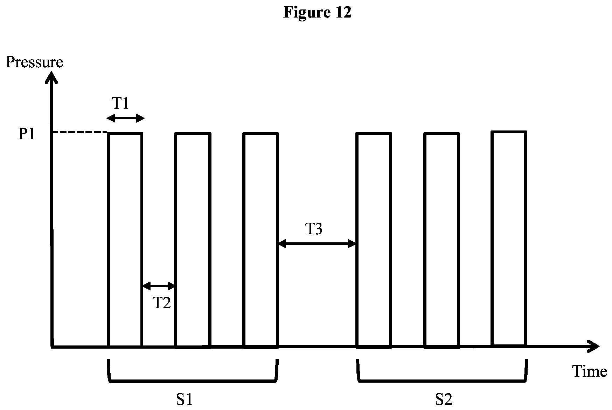

Fig. 12 shows a non-limiting example of such pulse sequence for use to induce vibration pattern in a treated portion of a plant. The graph depicts the pressure output (Y axis) as a function of time (X axis) of the air exiting the air flow applicators/apertures 203. The example depicted includes two pulse trains (S1 and S2) with a time separation T3, each pulse train with three pulses of duration T1 and gap T2. Vibration pattern can contain one or more pulse trains, with different time separations (T3) between the pulse trains, each pulse train with two or more pulses with a range of durations (T1) and gaps (T2). For example, in one embodiment, there can be three trains (S1, S2, S3), with time separation T3=0.5 seconds between the trains, each train with three pulses with gaps T2=0.1 seconds and each pulse lasting for T1=0.1 seconds.

The vibration of flowers in order to release pollen can be induced by air pressure bursts. This is a contact-less induction/generation of vibration of the flower(s) and/or flower truss or inflorescence in general, in order to induce pollination. Contact-less pollination can reduce chances of disease and virus transfer, and can reduce chances of damage to plant by improper contact. In contrast to non-directional air flow such as air blowers, the invention provides several advantages Blowers are much more energy consuming, non-pulsed so vibration frequency cannot be controlled, and pressure cannot be adjusted accurately. Due to the large air flow and non-directed and non-localized flow, air blowers can increase chances of spreading diseases, viruses and pests.

The amount of air, the burst numbers, duration and angle relative to the flowers should match the crop being pollinated, whether by user defined parameters or by pre-defined parameters following automatic detection of flower types, e.g. by vision and applied algorithm in the processing unit.

The air pulse sequence should have the following properties: 1) the full sequence should not be more than several seconds long in order to enable treatment of enough plants; 2) pulse length and distance/gap between pulses should enable vibration of flowers at required range of frequencies and amplitudes/magnitude; 3) pulse pressure and air flow rate should be kept to a minimum in order to conserve energy and/or pressure in tank; 4) aperture diameter/opening should not be too small that causes air to diverge and not reach flowers, or too large causing to deplete air too quickly. Valve releasing air to apertures should not be too far from apertures in order to conserve shape of pulse train, and not cause pulses to broaden before exiting aperture. Flow parameters should also be optimized in order not to damage the flowers and/or plant portion.

The plant treatment system/apparatus shown in figure 11 can be used for pollinating plants. One possible non-limiting method to pollinate self-pollinating inflorescence of a plant, by using the depicted system, can include the following steps:
1. Bringing vehicle 1001 adjacent to plant by methods described above;
2. View plant with imaging device 803;
3. Analyze images by processing unit 1016 or control system 107 to determine whether at least one flower on portion of plant viewed is ready for pollination. This can be done by a) comparing said image data with reference data showing development stage or growth phase indicative of pollination status of one or more flowers, e.g. a dataset with images of flowers ready for pollination, or b) by processing the image data to identify presence of a flower in the image(s) and readiness for pollination by identifying flower parameters indicative of existence or absence of pollination, such as colour and shape of one or more parts of the flower(s) or c) by utilizing trained artificial intelligence techniques (systems and/or methods);
4. Adjust position of applicator/apertures 203 to point at the required portion of plant by controlling mounts 204 as described above with regards to Fig. 8, by adjusting axis/line of fluid/air stream 811 according to image as seen by imaging devices 803 and/or 804 taking into account their respective offset from aperture 203;
5. Set vibration parameters (pressure, number and amplitude of pulses, each pulse duration and gaps between them) according to portion of plant to be vibrated as viewed by imaging devices and/or according to predefined values defined per portion of plant and/or distance to portion of plant and/or other parameters;
6. Release air pulse sequence to controllably vibrate the portion of plant.

In addition to the pollination method described above, once the air pulse sequence was delivered, the imaging device/camera can detect the flower vibration and if not meeting expected amplitude and/or frequency the vibration pattern can be adjusted in one of the following: pressure can be increased/decreased by controlling regulator, pulse duration and time gap between pulses can be changed in a train pulse, or the pulse number in a train pulse or the train pulse number, in order to change quantity, frequency and amplitude of vibrations. Pulse direction can also be changed. Instead of pointing to main inflorescence axis (rachis) or larger stem of plant in the aim of vibrating several inflorescences together and to vibrate all flowers together, pulses can be directed to individual flowers.

In order to prevent the need for pruning, the plant treatment system can include communication with an operator (by wireless communication or direct interface to the system) in order to predefine the exact plant being pollinated and focus the algorithm and improve its detection of targets as well as determine the amount of flowers that should be pollinated in each inflorescence or the total in each plant. Additionally, the system can be programmed to detect the plant by itself and have predefined parameters for the number of flowers to pollinate.

The plant treatment system/apparatus can use GPS or visual cues to record (from the set of cameras) the exact position of each visited plant and its flower status for later reference and reporting to farmer. The apparatus can also utilize signs/marks placed in the greenhouse (e.g. barcode sign per plant or row). The cameras can be hyperspectral or any type of camera, e.g. IR, and can have additional illumination in various wavelengths to enhance visibility and detection ability.

As described, the plant treatment system can be equipped with temperature, humidity and light sensors (Fig. 10, 1020a-c), or communicate with sensors placed in the farming area, and automatically determine whether to initiate or stop plant treatment (e.g. pollination) according to pre-defined parameters for each crop (following automatic detection of the crop being pollinated) or user defined settings.

When the plant treatment system/apparatus is equipped with environmental sensors providing environmental data about the plant surrounding, a method for pollinating inflorescences based on environmental data can include the following steps:
1. Collect environmental data from farming area;
2. if conditions fit vibration induced pollination then system can use treatment device(s) that utilizes air pulses;
3. if conditions do not fit such vibration induced pollination, plant microenvironment can be preconditioned:
   3a. if conditions are too dry, air can be humidified by the plant treatment device described in Fig. 4a for example, where one reservoir can contain water;
   3b. If conditions are too humid, relative humidity can be reduced by heating air applied to portion of plant treated by employing a heating element (as described in Fig. 7c);
4. if conditions do not allow release of pollen by vibration, and preconditioning components are not available, but pollen can attach to female organ, pollen can be locally and directionally applied by the treatment device described in Figs. 4a-c, where one reservoir contains pollen, and/or pollen can be administered by a contact based vibrating treatment device as described in Fig. 6.
5. if conditions do not allow pollen to attach to female organ, a treatment device such as described in figs. 4a-c can be used to spray plant hormones on flowers in order to induce growth of parthenocarpic fruits. Localized and directional administration of plant hormones is crucial since it is necessary to hit exact location of female organ in flowers, it reduces quantities both for saving purposes and since large amounts of hormones can damage plants.

The plant treatment system can selectively pollinate flowers. By visual cues from a set of imaging devices, or a combination of cameras and location determination by GPS or other method, the system will identify flowers on each plant, determine whether each flower is ready for pollination or was pollinated, determine whether a predefined number of flowers was already pollinated on the specific inflorescence. The system will then determine whether to pollinate a specific flower. Depending on the pollination method, the system will target only the flowers to be pollinated. If the pollination device utilizes vibration, as in Figure 3, selective pollination can be performed by placing the vibrating device adjacent to the flower and setting vibration amplitude such that adjacent flowers will not be pollinated. If the flowers are clustered, the vibration can be timed when the right number of flowers is ready for pollination by visually determining the state of all flowers in the cluster. The same can be performed with the air-pressure method (Fig. 4) and pollen or hormone spray method described above. This method is reversible, i.e. if a different number of flowers must be pollinated, the device can return to the plant and pollinate additional flowers.

In one embodiment of the plant treatment apparatus, described in Fig. 3, a vibrating element can be used for pollination of self-pollinating flowers that require vibration to release their pollen onto the female organ. The vibrating amplitude and frequency can change according to user definition or by pre-defined parameters following automatic detection of flower types by vision and algorithm in the processing unit. The placement of the vibrating element must also match the crop being pollinated. A set of cameras, on the vehicle and/or on the extension armIpost holding the plant treatment device and on the tip of the device will guide the system to place the vibrating element at the exact position of placement, whether at the base of each flower, at the rachis of the inflorescence, or at a branch holding several inflorescences.

Another embodiment of a plant treatment apparatus for pollinating flowers can be based on a brush that is placed on a distal tip of a manipulator arm (Fig. 6) and vibrating tip 301. The arm can guide the brush to a pollen reservoir on the vehicle or in places in the field in order to place pollen on the brush. The brush can then be guided, as is the vibrating tip, to pollinate flowers by placing the brush next to the female organ of the flower and gently rubbing (by vibration) the brush against the organ.

Referring back to Fig. 7, several examples of pollination inhibiting apparatuses are described. By placing such an apparatus on a vehicle as described in Figs. 10 and 11, by itself or together with other devices described above, portions of a plant can be damaged intentionally. For example, with a laser placed adjacent to imaging device, once the number of required flowers was pollinated, the rest can be intentionally damaged in order to prevent their pollination later. Depending on plant, the portions to be damaged in order to inhibit pollination may vary. For example, a self-pollinating flower can be inhibited by damaging either its male or female organs.

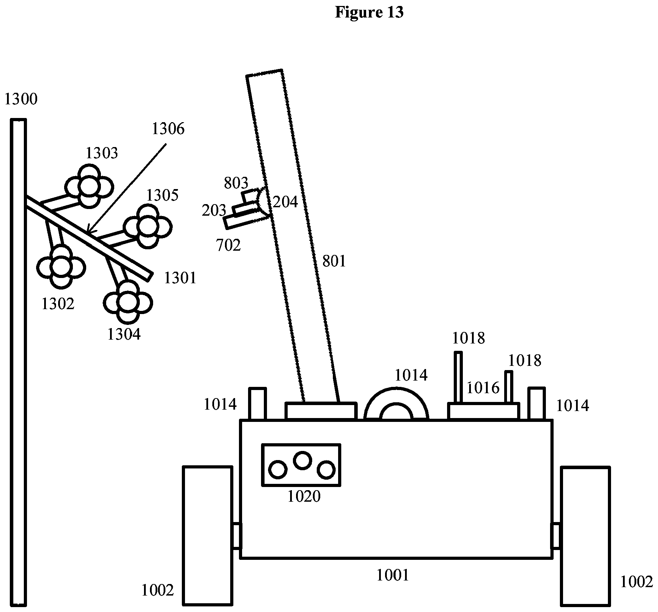

Fig. 13 depicts a non-limiting example of such a pollination inhibiting system. Portion of a plant 1300 is shown. Laser 702 is placed adjacent to air flow applicator/aperture 203, and both pointed by mount 204. The laser can be pointed to individual flowers 1302-1305, or to a location along the rachis 1301 that will damage all flowers beyond a point 1306 (i.e. flowers 1304 and 1305) and prevent from additional flowers to develop on the rachis. This prevents need for pruning since pollination can also occur spontaneously or naturally by wind and insects or movement of plant by farm personnel. Similarly, the damage can be done by hot air (from the heating mechanism described above, set to temperatures which can be pre-defined per type of plant, flower, flower status and/or environmental conditions, as detected by system's imaging devices and/or environmental sensors and are analyzed by processing unit 1016 or control system 107), or by placing a damaging material in the tank and spraying the flowers accurately from close range without damaging other flowers. This requires an apparatus for directed localized substance/fluid delivery as described in Fig. 4. Both directed and localized substance delivery and directed and localized hot air delivery, together with control of quantity of substance or heat of hot air stream, can minimize damage to surrounding flowers and portion of plant or surrounding plants. Similarly, laser energy can be minimized to pre-defined values per type of plant, flower, flower status and/or environmental conditions, as well as setting laser spot size on target to several millimeters or less (depending on exact inflorescence targeted) in order to prevent damage to surrounding portions of plant, since flower organs to be targeted and rachis of inflorescence are on the order of several millimeters.

With multiple cameras existing on the various mounts and posts, each plant can be viewed from many angles and heights up close. This enables detecting pest or diseases. The tank 401 described above (shown in Figs. 4a-c) can contain other treatment materials (e.g. pesticide) and the air pressure mechanism can be used to spray it or other materials for local and efficient treatment of pests, diseases, fungi etc. The system can notify user on the finding and its treatment, and in subsequent visits, since the system registers location of each plant, the status of the disease or pest can be updated in order to ensure that the problem was treated. When system is as described in Fig. 4c, i.e. consisting of several tanks, several materials can be placed on the same vehicle, and several diseases or pests detected can be treated.

## Claims

1. A plant treatment system (100) comprising:
a plant treatment apparatus (102) comprising:
one or more plant treatment devices (103) being configured and operable to apply treatment to at least a portion of the plant, wherein one or more devices of said one or more plant treatment devices being configured and operable to apply the treatment by controllably inducing a vibration pattern in the at least portion of the plant, said vibration pattern being costumed to a desired kind of the treatment to be applied; and
a sensing system (104) comprising one or more sensors (105) configured and operable to provide sensing signals indicative of a condition of said at least portion of the plant, said one or more sensors comprising an optical sensor (105a) configured and operable to provide the sensing signals indicative of image data of said at least portion of the plant; and
a control system configured and operable for data communication with said plant treatment apparatus, to receive and process the sensing signals produced by the sensing system, the processing of the sensing signals comprising determining a condition of said at least portion of the plant, defining parameters of the vibration pattern and operating said at least one plant treatment device to induce the vibration pattern corresponding to the desired kind of the treatment to be applied to said at least portion of the plant;
the plant treatment system being **characterized in that**
said plant treatment apparatus comprises one or more fluid flow channels (106,
205) and one or more fluid flow applicators (203), and
at least a first device (103a) of said one or more plant treatment devices is configured and operable to induce the vibration pattern by generating, via said one or more fluid flow channels and one or more fluid flow applicators, an air flow having a predetermined flow profile, towards said at least portion of the plant, said predetermined flow profile of the air flow being a directional and targeted air stream that can be directed towards and induces the vibration pattern in specific one or more regions in said at least portion of the plant.

2. The plant treatment system according to claim 1,
wherein at least a second device (103b) of said one or more plant treatment devices comprises a vibrating element (302) being configured and operable to contact said at least portion of the plant while vibrating to thereby controllably induce said vibration pattern in the at least portion of the plant.

3. The plant treatment system according to any one of the preceding claims, wherein said desired kind of treatment is pollination, wherein said first or second plant treatment device is configured and operable as a plant pollination device, said control system is configured and operable to process the sensing signals and, upon determining that a flower within said at least portion of the plant is to be pollinated, generate corresponding operational data for said first or second plant treatment device such that said induced vibration pattern is configured to cause pollination of at least one flower within said at least portion of the plant.

4. The plant treatment system according to any one of the preceding claims, wherein said one or more plant treatment devices comprise a substance delivery device (103c) configured and operable to locally deliver or spray at least one of a treatment substance or pollen onto one or more regions of said at least portion of the plant, said treatment substance comprising at least one of the following: a medicament for treating plant disease, a plant hormone inducing plant growth, a pesticide that kills pests, or a plant damaging substance that prevents growth and/or pollination.

5. The plant treatment system according to claim 4, wherein said substance delivery device utilizes said one or more fluid flow channels and said one or more fluid flow applicators to deliver or spray the treatment substance or the pollen.

6. The plant treatment system according to any one of the preceding claims, wherein said control system is configured and operable to define at least one of the following parameters of the vibration pattern: frequency, amplitude and duration of the vibration pattern.

7. The plant treatment system according to any one of the preceding claims, wherein said control system is configured and operable to define at least one of the following parameters of the vibration pattern: number of train pulses of air, time gap between train pulses, number of pulses in each train pulse, time gap between two pulses in each train pulse, amplitude of pressure in each pulse, duration of each pulse.

8. The plant treatment system according to any one of the preceding claims, wherein said at least portion of the plant being treated is located within a field of view of the optical sensor, said optical sensor and the fluid flow channel are configured with a predetermined fixed relative orientation comprising at least one of an offset or angular difference between axis of line of sight of the optical sensor and axis of propagation of the directional and targeted air stream.

9. The plant treatment system according to claim 8, having at least one of the following configurations:
a) a light collecting plane of said optical sensor is located adjacently to a fluid exit aperture of said fluid flow applicator, and
b) said optical sensor and a fluid exit aperture of said fluid flow applicator are fixedly attached.

10. The plant treatment system according to any one of the preceding claims, wherein said one or more plant treatment devices further comprise a pollen transport device (103d) configured and operable to collect pollen from a container on vehicle or in farming area and deliver the collected pollen to a pistil of at least one flower within said at least portion of the plant.

11. The plant treatment system according to claim 10, wherein said pollen transport device has a patterned surface configured to adhere the pollen being collected to said surface.

12. The plant treatment system according to any one of the preceding claims, wherein said sensing system further comprises one or more environmental sensors configured and operable to provide the sensing signals indicative of one or more environmental conditions in a vicinity of said at least portion of the plant.

13. The plant treatment system according to claim 12, wherein said one or more plant treatment devices further comprise an environment conditioning device being configured and operable to modify at least one of temperature and humidity of a surrounding of said at least portion of the plant.

14. The plant treatment system according to claim 13, wherein said environment conditioning device utilizes said one or more fluid flow channels and said one or more fluid flow applicators to modify the temperature or humidity by air flow.

15. The plant treatment system according to any one of the claims 4 to 14, wherein said sensing system is **characterized by** at least one of the following: a) the sensing system comprises one or more environmental sensors configured and operable to provide the sensing signals indicative of one or more environmental conditions in a vicinity of said at least portion of the plant, wherein said sensing signals are indicative of unfavorable conditions for pollination, and wherein said control system generates operational data for said substance delivery device to deliver or spray a hormone that induces parthenocarpic fruit growth, and b) the sensing system provides said sensing signals being indicative of a disease of said at least portion of the plant or pest in a surrounding of or on said at least portion of the plant, and wherein said control system generates operational data for said substance delivery device to deliver or spray a medicament or a pesticide respectively.

16. The plant treatment system according to any one of the preceding claims, wherein said plant treatment apparatus further comprises at least one of a sterilization, cleaning or disinfecting assembly (911) configured and operable to respectively sterilize, clean or disinfect said one or more plant treatment devices, the assembly comprising at least one of the following: a hot air blower (912), a cleaning material applicator and a cleaning or disinfecting or sterilizing material sprayer.

17. The plant treatment system according to any one of the preceding claims, wherein said plant treatment apparatus comprises a navigation and tracking assembly (1000) configured and operable to bring the plant treatment apparatus to a vicinity of said at least portion of the plant to thereby enable treating said at least portion of the plant by the plant treatment system, the plant treatment system being **characterized in** at least one of the following:
a) said navigation and tracking assembly comprises a robotic arm (901) carrying said plant treatment apparatus, and wherein said control system is configured and operable to controllably move the robotic arm in three dimensions,
b) said navigation and tracking assembly comprises a ground vehicle (1001) configured and operable to controllably transport the plant treatment apparatus to the vicinity of said at least portion of the plant,
c) said navigation and tracking assembly comprises at least one of the following: one or more optical sensors (1014), and a positioning sensor,
d) said navigation and tracking assembly comprises a robotic arm carrying said plant treatment apparatus and an inertial moment unit configured and operable to determine spatial movement path of the robotic arm to thereby optimize plant treatment process, time and energy.

18. The plant treatment system according to any one of the preceding claims, wherein said control system is **characterized by** at least one of the following:
a) the control system is configured and operable to determine, based on said sensing signals, whether at least one flower on said portion of the plant is ready for pollination, by comparing said sensing signals with reference data comprising images of flowers ready for pollination, or by processing said image data to identify presence of a flower in the image(s) and identify readiness of the flower(s) for pollination by identifying flower parameters indicative of existence or absence of pollination or by utilizing trained artificial intelligence, and
b) the control system is configured and operable to analyze the sensing signals from at least the optical sensor and determine a condition of said at least portion of the plant while being treated and after the treatment, and generate corresponding feedback data, enabling decision making about modification of at least one parameter of the treatment affecting the vibration pattern induced in the at least portion of the plant.

19. The plant treatment system according to any one of the preceding claims, wherein said one or more plant treatment devices further comprise a pollination inhibiting device (103e) configured and operable to prevent pollination to occur to one or more flowers or prevent growth and blossoming of additional flowers within said at least portion of the plant, while minimizing damage to nearby parts of the plant, the pollination inhibiting device being **characterized by** at least one of the following:
a) the pollination inhibiting device comprises a laser device (702, 701) configured and operable to irradiate said at least portion of the plant with predetermined laser parameters to thereby damage said at least portion of the plant,
b) the pollination inhibiting device is configured and operable to generate, via said one or more fluid flow channels, an air flow having a predetermined high temperature while maintaining air flow directionality by controlling size of air flow exit, to damage one or more regions of said at least portion of the plant and prevent pollination to occur to one or more flowers or prevent growth and blossoming of additional flowers within said at least portion of the plant, while minimizing damage to nearby parts of the plant.

20. A plant treatment apparatus comprising:
one or more plant treatment devices (103, 103e), being configured and operable to apply treatment to at least a portion of a plant;
wherein one or more devices of said one or more plant treatment devices are configured and operable to apply the treatment by controllably inducing a vibration pattern in the at least portion of the plant, said vibration pattern being costumed to a desired kind of the treatment to be applied;
a sensing system (104) comprising one or more sensors (105) configured and operable to provide sensing signals indicative of a condition of said at least portion of the plant, said one or more sensors comprising an optical sensor (105a) configured and operable to provide the sensing signals indicative of image data of said at least portion of the plant; and
a communication utility for data communication with a control system to transmit the sensing signals to the control system and receive from the control system operational data for said at least one plant treatment device induce the vibration pattern corresponding to the desired kind of the treatment to be applied to said at least portion of the plant;
the plant treatment apparatus being **characterized in that**:
said plant treatment apparatus comprises one or more fluid flow channels (106, 205) and one or more fluid flow applicators (203), and
at least a first device (103a) of said one or more plant treatment devices is configured and operable to induce the vibration pattern by generating, via said one or more fluid flow channels and one or more fluid flow applicators, an air flow having a predetermined flow profile, towards said at least portion of the plant, said predetermined flow profile of the air flow being a directional and targeted air stream that can be directed towards and induces the vibration pattern in specific one or more regions in said at least portion of the plant.

21. The plant treatment apparatus according to claim 20, wherein said one or more plant treatment devices (103, 103e) are configured and operable to cause targeted damage to at least a part of said at least portion of the plant by blowing, via one or more of said fluid flow channels (106, 205) and said one or more fluid flow applicators (203), air of predetermined high temperature and flow profile on the at least part of the at least portion of the plant, said operational data for said at least one plant treatment device being configured to cause the targeted damage to said at least part of the at least portion of the plant based on following one or more conditions of said at least portion of the plant: disease, pest, flower status, number of flowers, number of flowers that have been already pollinated.

22. The plant treatment apparatus according to claim 20 or 21, wherein said one or more plant treatment devices further comprise at least one of the following: laser (702) of predetermined parameters of intensity and/or wavelength, and substance delivery.

23. The plant treatment apparatus according to any one of claims 20 to 22, wherein said plant treatment apparatus further comprises a navigation and tracking assembly (1000) configured and operable to bring the plant treatment apparatus to a vicinity of said at least portion of the plant to thereby enable treating said at least portion of the plant by the plant treatment device, said navigation and tracking assembly comprises one of more of the following:
a) a robotic arm (901) carrying said plant treatment apparatus and being configured and operable to move in three dimensions,
b) a ground vehicle (1001) configured and operable to controllably transport the plant treatment apparatus to the vicinity of said at least portion of the plant,
c) at least one of the following: one or more optical sensors (1014), and a positioning sensor,
d) a robotic arm carrying said plant treatment apparatus and an inertial moment unit configured and operable to determine spatial movement path of the robotic arm to thereby optimize plant treatment process, time and energy.

24. The plant treatment apparatus according to any one of claims 20 to 23, wherein said at least part of the at least portion of the plant is a single flower or a region in a single flower.

25. A method for plant treatment comprising:
- acquiring sensing data comprising image data of at least a portion of a plant;
- analyzing said sensing data to determine whether one or more flowers on said at least portion of the plant satisfy one of the following conditions: (i) said one or more flowers are ready for pollination, or (ii) said one or more flowers have been pollinated;
- selectively performing one of the following:
(i) upon detecting one or more flowers ready for pollination, defining parameters of vibration costumed to induce a desired vibration pattern in said one or more flowers ready for pollination, the vibration pattern being induced by application of air flow having a predetermined flow profile being a directional and targeted air stream directed towards the one or more flowers ready for pollination, and generating operational data indicative thereof; and applying said operational data to said one or more flowers ready for pollination to thereby pollinate said one or more flowers ready for pollination; and
(ii) upon determining that the one or more flowers have been pollinated, inhibiting pollination of other flower(s) or preventing growth and blossoming of other flower(s) on said at least a portion of the plant, by directing a fluid stream of predetermined temperature, velocity and spatial profiles to at least part of said at least portion of the plant.

## Patentansprüche

1. Pflanzenbehandlungssystem (100), umfassend:
Pflanzenbehandlungsgerät (102), umfassend:
eine oder mehrere Pflanzenbehandlungsvorrichtungen (103), die konfiguriert sind und betrieben werden können, um eine Behandlung auf mindestens einen Abschnitt der Pflanze anzuwenden, wobei eine oder mehrere Vorrichtungen der einen oder der mehreren Pflanzenbehandlungsvorrichtungen konfiguriert sind und betrieben werden können, um sie Behandlung durch kontrolliertes Induzieren eines Vibrationsmusters in dem mindestens einen Abschnitt der Pflanze anzuwenden, wobei das Vibrationsmuster auf eine gewünschte Art der anzuwendenden Behandlung abgestimmt ist; und
ein Erfassungssystem (104), umfassend einen oder mehrere Sensoren (105), die konfiguriert sind und betrieben werden können, um Erfassungssignale bereitzustellen, die indikativ für einen Zustand des mindestens einen Abschnitts der Pflanze sind, der eine oder die mehreren Sensoren umfassend einen optischen Sensor (105a), der konfiguriert ist und betrieben werden kann, um die Erfassungssignale bereitzustellen, die indikativ für Bilddaten des mindestens einen Abschnitts der Pflanze sind; und
ein Steuersystem, das für eine Datenkommunikation mit dem Pflanzenbehandlungsgerät konfiguriert ist und betrieben werden kann, um die von dem Erfassungssystem erzeugten Erfassungssignale zu empfangen und zu verarbeiten, die Verarbeitung der Erfassungssignale umfassend ein Bestimmen eines Zustands des mindestens einen Abschnitts der Pflanze, ein Definieren von Parametern des Vibrationsmusters und ein Betreiben der mindestens einen Pflanzenbehandlungsvorrichtung, um das Vibrationsmuster zu erzeugen, das der gewünschten Art der Behandlung entspricht, die auf den mindestens einen Abschnitt der Pflanze angewendet werden soll;
wobei das Pflanzenbehandlungssystem **dadurch gekennzeichnet ist, dass**
das Pflanzenbehandlungsgerät einen oder mehrere Fluidströmungskanäle (106, 205)
und einen oder mehrere Fluidströmungsapplikatoren (203) umfasst, und
mindestens eine erste Vorrichtung (103a) der einen oder mehreren Pflanzenbehandlungsvorrichtungen konfiguriert ist und betrieben werden kann, um das Vibrationsmuster zu induzieren, indem sie über den einen oder die mehreren Fluidströmungskanäle und den einen oder die mehreren Fluidströmungsapplikatoren einen Luftstrom mit einem vorbestimmten Strömungsprofil in Richtung des mindestens einen Abschnitts der Pflanze erzeugt, wobei das vorbestimmte Strömungsprofil des Luftstroms ein gerichteter und gezielter Luftstrom ist, der in Richtung eines oder mehrerer spezifischer Bereiche in dem mindestens einen Abschnitt der Pflanze gerichtet werden kann und das Vibrationsmuster dort induziert.

2. Pflanzenbehandlungssystem nach Anspruch 1,
wobei mindestens eine zweite Vorrichtung (103b) der einen oder mehreren Pflanzenbehandlungsvorrichtungen ein Vibrationselement (302) umfasst, das konfiguriert ist und betrieben werden kann, um den mindestens einen Abschnitt der Pflanze zu berühren, während es vibriert, um dadurch das Vibrationsmuster in dem mindestens einen Abschnitt der Pflanze steuerbar zu induzieren.

3. Pflanzenbehandlungssystem nach einem der vorherigen Ansprüche, wobei die gewünschte Art der Behandlung eine Bestäubung ist, wobei die erste oder zweite Pflanzenbehandlungsvorrichtung als Pflanzenbestäubungsvorrichtung konfiguriert ist und betrieben werden kann, wobei das Steuersystem konfiguriert ist und betrieben werden kann, um die Erfassungssignale zu verarbeiten und bei einem Bestimmen, dass eine Blüte innerhalb des mindestens einen Abschnitts der Pflanze bestäubt werden soll, entsprechende Betriebsdaten für die erste oder zweite Pflanzenbehandlungsvorrichtung zu erzeugen, sodass das induzierte Vibrationsmuster konfiguriert ist, um eine Bestäubung mindestens einer Blüte innerhalb des mindestens einen Abschnitts der Pflanze zu bewirken.

4. Pflanzenbehandlungssystem nach einem der vorherigen Ansprüche, wobei die eine oder die mehreren Pflanzenbehandlungsvorrichtungen eine Substanzzufuhrvorrichtung (103c) umfassen, die konfiguriert ist und betrieben werden kann, um örtlich eine Behandlungssubstanz und/oder Pollen auf einen oder mehrere Bereiche des mindestens einen Abschnitts der Pflanze zuzuführen oder zu sprühen, wobei die Behandlungssubstanz mindestens eines von folgenden umfasst: ein Medikament zum Behandeln von Pflanzenkrankheit, ein Pflanzenhormon, das Pflanzenwachstum induziert, ein Pestizid, das Schädlinge abtötet, oder eine pflanzenschädigende Substanz, die Wachstum und/oder Bestäubung verhindert.

5. Pflanzenbehandlungssystem nach Anspruch 4, wobei die Substanzzufuhrvorrichtung den einen oder die mehreren Fluidströmungskanäle und den einen oder die mehreren Fluidströmungsapplikatoren verwendet, um die Behandlungssubstanz oder den Pollen zuzuführen oder zu sprühen.

6. Pflanzenbehandlungssystem nach einem der vorherigen Ansprüche, wobei das Steuersystem konfiguriert ist und betrieben werden kann, um mindestens einen der folgenden Parameter des Vibrationsmusters definiert: Frequenz, Amplitude und Dauer des Vibrationsmusters.

7. Pflanzenbehandlungssystem nach einem der vorherigen Ansprüche, wobei das Steuersystem konfiguriert ist und betrieben werden kann, um mindestens einen der folgenden Parameter des Vibrationsmusters zu definieren: Anzahl von Zugimpulsen der Luft, Zeitabstand zwischen Zugimpulsen, Anzahl von Impulsen in jedem Zugimpuls, Zeitabstand zwischen zwei Impulsen in jedem Zugimpuls, Amplitude des Drucks in jedem Impuls, Dauer von jedem Impuls.

8. Pflanzenbehandlungssystem nach einem der vorherigen Ansprüche, wobei sich der mindestens eine Abschnitt der zu behandelnden Pflanze in einem Sichtfeld des optischen Sensors befindet und der optische Sensor und der Fluidströmungskanal mit einer vorbestimmten festen relativen Ausrichtung konfiguriert sind, die mindestens einen Versatz oder eine Winkeldifferenz zwischen einer Sichtlinienachse des optischen Sensors und der Ausbreitungsachse des gerichteten und gezielten Luftstroms umfasst.

9. Pflanzenbehandlungssystem nach Anspruch 8, das mindestens eine der folgenden Konfigurationen aufweist:
a) eine Lichtsammelebene des optischen Sensors ist benachbart zu einer Flüssigkeitsaustrittsöffnung des Flüssigkeitsstromapplikators angeordnet, und
b) der optische Sensor und eine Flüssigkeitsaustrittsöffnung des Flüssigkeitsstromapplikators sind fest verbunden.

10. Pflanzenbehandlungssystem nach einem der vorherigen Ansprüche, wobei die eine oder die mehreren Pflanzenbehandlungsvorrichtungen ferner eine Pollentransportvorrichtung (103d) umfassen, die konfiguriert ist und betrieben werden kann, um Pollen aus einem Behälter auf einem Fahrzeug oder in einem landwirtschaftlichen Gebiet zu sammeln und den gesammelten Pollen an einen Stempel mindestens einer Blüte innerhalb des mindestens einen Abschnitts der Pflanze zuzuführen.

11. Pflanzenbehandlungssystem nach Anspruch 10, wobei die Pollentransportvorrichtung eine strukturierte Oberfläche aufweist, die konfiguriert ist, um den gesammelten Pollen an der Oberfläche anzuhaften.

12. Pflanzenbehandlungssystem nach einem der vorherigen Ansprüche, wobei das Erfassungssystem ferner einen oder mehrere Umgebungssensoren umfasst, die konfiguriert sind und betrieben werden können, um die Erfassungssignale bereitzustellen, die indikativ für einen oder mehrere Umgebungsbedingungen in der Nähe des mindestens einen Abschnitts der Pflanze sind.

13. Pflanzenbehandlungssystem nach Anspruch 12, wobei die eine oder die mehreren Pflanzenbehandlungsvorrichtungen ferner eine Umgebungskonditionierungsvorrichtung umfassen, die konfiguriert ist und betrieben werden kann, um mindestens eine von Temperatur und Feuchtigkeit der Umgebung des mindestens einen Abschnitts der Pflanze zu verändern.

14. Pflanzenbehandlungssystem nach Anspruch 13, wobei die Umgebungskonditionierungsvorrichtung den einen oder die mehreren Fluidströmungskanäle und den einen oder die mehreren Fluidströmungsapplikatoren verwendet, um die Temperatur oder Feuchtigkeit durch Luftströmung zu verändern.

15. Pflanzenbehandlungssystem nach einem der Ansprüche 4 bis 14, wobei das Erfassungssystem durch mindestens eines der folgenden gekennzeichnet ist: a) das Erfassungssystem umfasst einen oder mehrere Umweltsensoren, die konfiguriert sind und betrieben werden können, um die Erfassungssignale bereitzustellen, die indikativ für eine oder mehrere Umweltbedingungen in der Nähe des mindestens einen Abschnitts der Pflanze sind, wobei die Erfassungssignale indikativ für ungünstige Bedingungen für eine Bestäubung sind, und wobei das Steuersystem Betriebsdaten für die Substanzzufuhrvorrichtung erzeugt, um ein Hormon zuzuführen oder zu sprühen, das ein Wachstum parthenokarper Früchte induziert, und b) das Erfassungssystem stellt Erfassungssignale bereit, die indikativ für eine Krankheit des mindestens einen Abschnitts der Pflanze oder einen Schädling in der Umgebung des mindestens einen Abschnitts der Pflanze oder darauf sind, und wobei das Steuersystem Betriebsdaten für die Substanzzufuhrvorrichtung erzeugt, um ein Medikament bzw. ein Pestizid zuzuführen oder zu sprühen.

16. Pflanzenbehandlungssystem nach einem der vorherigen Ansprüche, wobei das Pflanzenbehandlungsgerätferner mindestens eines von einer Sterilisations-, Reinigungs- oder Desinfektionsanordnung (911) umfasst, die konfiguriert ist und betrieben werden kann, um die eine oder mehreren Pflanzenbehandlungsvorrichtungen zu sterilisieren, zu reinigen oder zu desinfizieren, die Anordnung umfassend mindestens eines von folgenden: ein Heißluftgebläse (912), einen Reinigungsmaterialapplikator und einen Sprüher für Reinigungs- oder Desinfektions- oder Sterilisationsmaterial.

17. Pflanzenbehandlungssystem nach einem der vorherigen Ansprüche, wobei das Pflanzenbehandlungsgerät eine Navigations- und Verfolgungsanordnung (1000) umfasst, die konfiguriert ist und betrieben werden kann, um die Pflanzenbehandlungsvorrichtung in die Nähe des mindestens einen Abschnitts der Pflanze zu bringen, um dadurch eine Behandlung des mindestens einen Abschnitts der Pflanze durch das Pflanzenbehandlungssystem zu ermöglichen, wobei das Pflanzenbehandlungssystem durch mindestens eines von folgenden gekennzeichnet ist:
a) die Navigations- und Verfolgungsanordnung umfasst einen Roboterarm (901), der das Pflanzenbehandlungsgerät trägt, und wobei das Steuersystem konfiguriert ist und betrieben werden kann, um den Roboterarm steuerbar in drei Dimensionen zu bewegen,
b) die Navigations- und Verfolgungsanordnung umfasst ein Bodenfahrzeug (1001), das konfiguriert ist und betrieben werden kann, um das Pflanzenbehandlungsgerät steuerbar in die Nähe des mindestens einen Abschnitts der Pflanze zu transportieren,
c) die Navigations- und Verfolgungsanordnung umfasst mindestens eines von folgenden: einen oder mehrere optische Sensoren (1014) und einen Positionierungssensor,
d) die Navigations- und Verfolgungsanordnung umfasst einen Roboterarm, der das Pflanzenbehandlungsgerät trägt, und eine Trägheitsmomenteinheit, die konfiguriert ist und betrieben werden kann, um eine räumliche Bewegungsbahn des Roboterarms zu bestimmen, um dadurch Prozess, Zeit und Energie der Pflanzenbehandlung zu optimieren.

18. Pflanzenbehandlungssystem nach einem der vorherigen Ansprüche, wobei das Steuersystem durch mindestens eines der folgenden gekennzeichnet ist:
a) das Steuersystem ist konfiguriert und kann betrieben werden, um basierend auf den Erfassungssignalen zu bestimmen, ob mindestens eine Blüte auf dem Abschnitt der Pflanze zur Bestäubung bereit ist, indem es die Erfassungssignale mit Referenzdaten vergleicht, die Bilder von zur Bestäubung bereiten Blüten umfassen, oder indem es die Bilddaten verarbeitet, um das Vorhandensein einer Blüte in dem Bild/den Bildern zu identifizieren und die Bereitschaft der Blüte(n) zur Bestäubung zu identifizieren, indem es Blütenparameter identifiziert, die indikativ für ein Vorhandensein oder eine Abwesenheit einer Bestäubung sind, oder indem es eine trainierte künstliche Intelligenz verwendet, und
b) das Steuersystem konfiguriert ist und betrieben werden kann, um die Messsignale von mindestens dem optischen Sensor zu analysieren und einen Zustand des mindestens einen Abschnitts der Pflanze während der Behandlung und nach der Behandlung zu bestimmen und entsprechende Rückmeldedaten zu erzeugen, die eine Entscheidungsfindung über eine Änderung mindestens eines Parameters der Behandlung ermöglichen, der das in dem mindestens einen Abschnitt der Pflanze induzierte Vibrationsmuster beeinflusst.

19. Pflanzenbehandlungssystem nach einem der vorherigen Ansprüche, wobei die eine oder die mehreren Pflanzenbehandlungsvorrichtungen ferner eine Bestäubungshemmungsvorrichtung (103e) umfassen, die konfiguriert ist und betrieben werden kann, um ein Auftreten einer Bestäubung für eine oder mehrere Blüten zu verhindern oder ein Wachstum und Blühen zusätzlicher Blüten innerhalb des mindestens einen Abschnitts der Pflanze zu verhindern, während eine Schädigung für nahe gelegene Teile der Pflanze minimiert wird, wobei die Bestäubungshemmungsvorrichtung durch mindestens eines der folgenden gekennzeichnet ist:
a) die Bestäubungshemmungsvorrichtung umfasst eine Laservorrichtung (702, 701), die konfiguriert ist und betrieben werden kann, um den mindestens einen Abschnitt der Pflanze mit vorbestimmten Laserparametern zu bestrahlen, um dadurch den mindestens einen Abschnitt der Pflanze zu schädigen,
b) die Bestäubungshemmungsvorrichtung ist konfiguriert und kann betrieben werden, um über den einen oder die mehreren Fluidströmungskanäle einen Luftstrom mit einer vorbestimmten hohen Temperatur zu erzeugen, während sie die Luftstromrichtung aufrechtzuerhalten, indem sie die Größe des Luftstromaustritts steuert, um einen oder mehrere Bereiche des mindestens einen Abschnitts der Pflanze zu beschädigen und zu verhindern, dass eine oder mehrere Blüten bestäubt werden, oder um ein Wachstum und Blühen zusätzlicher Blüten in dem mindestens einen Abschnitt der Pflanze zu verhindern, während eine Schädigung benachbarter Teile der Pflanze minimiert wird.

20. Pflanzenbehandlungsgerät, umfassend:
eine oder mehrere Pflanzenbehandlungsvorrichtungen (103, 103e), die konfiguriert sind und betrieben werden können, um mindestens einen Abschnitt einer Pflanze zu behandeln;
wobei eine oder mehrere Vorrichtungen der einen oder mehreren Pflanzenbehandlungsvorrichtungen konfiguriert sind und betrieben werden können, um die Behandlung durch kontrolliertes Induzieren eines Vibrationsmusters in dem mindestens einen Abschnitt der Pflanze anzuwenden, wobei das Vibrationsmuster auf eine gewünschte Art der anzuwendenden Behandlung abgestimmt ist;
ein Erfassungssystem (104), umfassend einen oder mehrere Sensoren (105), die konfiguriert sind und betrieben werden können, um Erfassungssignale bereitzustellen, die indikativ für einen Zustand des mindestens einen Abschnitts der Pflanze sind, der eine oder die mehreren Sensoren umfassend einen optischen Sensor (105a), der konfiguriert ist und betrieben werden kann, um die Erfassungssignale bereitzustellen, die indikativ für Bilddaten des mindestens einen Abschnitts der Pflanze sind; und
eine Kommunikationseinrichtung für Datenkommunikation mit einem Steuersystem, um die Erfassungssignale an das Steuersystem zu übertragen und von dem Steuersystem Betriebsdaten für die mindestens eine Pflanzenbehandlungsvorrichtung zu empfangen, um das Vibrationsmuster entsprechend der gewünschten Art der auf den mindestens einen Abschnitt der Pflanze anzuwendenden Behandlung zu induzieren;
wobei das Pflanzenbehandlungsgerät **dadurch gekennzeichnet ist, dass** das Pflanzenbehandlungsgerät einen oder mehrere Fluidströmungskanäle (106, 205) und einen oder mehrere Fluidströmungsapplikatoren (203) umfasst, und
mindestens eine erste Vorrichtung (103a) der einen oder mehreren Pflanzenbehandlungsvorrichtungen konfiguriert ist und betrieben werden kann, um das Vibrationsmuster zu induzieren, indem sie über den einen oder die mehreren Fluidströmungskanäle und den einen oder die mehreren Fluidströmungsapplikatoren einen Luftstrom mit einem vorbestimmten Strömungsprofil in Richtung des mindestens einen Abschnitts der Pflanze erzeugt, wobei das vorbestimmte Strömungsprofil des Luftstroms ein gerichteter und gezielter Luftstrom ist, der in Richtung eines oder mehrerer spezifischer Bereiche in dem mindestens einen Abschnitt der Pflanze gerichtet werden kann und das Vibrationsmuster dort induziert.

21. Pflanzenbehandlungsgerät nach Anspruch 20, wobei die eine oder die mehreren Pflanzenbehandlungsvorrichtungen (103, 103e) konfiguriert sind und betrieben werden können, um gezielte Schäden an mindestens einem Teil des mindestens einen Abschnitts der Pflanze zu verursachen, indem sie über einen oder mehrere der Fluidströmungskanäle (106, 205) und den einen oder die mehreren Fluidströmungsapplikatoren (203) Luft mit einer vorbestimmten hohen Temperatur und einem vorbestimmten Strömungsprofil auf den mindestens einen Abschnitt des mindestens einen Abschnitts der Pflanze bläst, wobei die Betriebsdaten für die mindestens eine Pflanzenbehandlungsvorrichtung konfiguriert sind, um den gezielten Schaden an dem mindestens einen Abschnitt des mindestens einen Abschnitts der Pflanze basierend auf folgender einen oder folgenden mehreren Bedingungen des mindestens einen Abschnitts der Pflanze zu verursachen: Krankheit, Schädling, Blütenstatus, Anzahl Blüten, Anzahl bereits bestäubter Blüten.

22. Pflanzenbehandlungsgerät nach Anspruch 20 oder 21, wobei die eine oder die mehreren Pflanzenbehandlungsvorrichtungen ferner mindestens eines der folgenden umfassen: Laser (702) mit vorbestimmten Intensitäts- und/oder Wellenlängenparametern und Substanzzuführung.

23. Pflanzenbehandlungsgerät nach einem der vorherigen Ansprüche 20 bis 22, wobei das Pflanzenbehandlungsgerät eine Navigations- und Verfolgungsanordnung (1000) umfasst, die konfiguriert ist und betrieben werden kann, um das Pflanzenbehandlungsgerät in die Nähe des mindestens einen Abschnitts der Pflanze zu bringen, um dadurch eine Behandlung des mindestens einen Abschnitts der Pflanze durch die Pflanzenbehandlungsvorrichtung zu ermöglichen, wobei die Navigations- und Verfolgungsanordnung eines oder mehrere der folgenden umfasst:
a) einen Roboterarm (901), der das Pflanzenbehandlungsgerät trägt und konfiguriert ist und betrieben werden kann, um sich in drei Dimensionen zu bewegen,
b) ein Bodenfahrzeug (1001), das konfiguriert ist und betrieben werden kann, um das Pflanzenbehandlungsgerät steuerbar in die Nähe des mindestens einen Abschnitts der Pflanze zu transportieren,
c) mindestens eines der folgenden: einen oder mehrere optische Sensoren (1014) und einen Positionierungssensor,
d) einen Roboterarm, der das Pflanzenbehandlungsgerät trägt, und eine Trägheitsmomenteinheit, die konfiguriert ist und betrieben werden kann, um eine räumliche Bewegungsbahn des Roboterarms zu bestimmen, um dadurch Prozess, Zeit und Energie der Pflanzenbehandlung zu optimieren.

24. Pflanzenbehandlungsgerät nach einem der Ansprüche 20 bis 23, wobei der mindestens eine Teil des mindestens einen Abschnitts der Pflanze eine einzelne Blüte oder ein Bereich in einer einzelnen Blüte ist.

25. Verfahren zur Pflanzenbehandlung, umfassend:
- Aufnehmen von Erfassungsdaten, umfassend Bilddaten von mindestens einem Abschnitt einer Pflanze;
- Analysieren der Erfassungsdaten, um zu bestimmen, ob eine oder mehrere Blüten auf dem mindestens einen Abschnitt der Pflanze eine der folgenden Bedingungen erfüllen: (i) die eine oder mehreren Blüten sind zur Bestäubung bereit oder (ii) die eine oder mehreren Blüten sind bestäubt worden;
- selektives Ausführen eines von folgenden:
(i) beim Erfassen einer oder mehrerer bestäubungsbereiter Blumen, Definieren von Vibrationsparametern, die angepasst sind, um ein gewünschtes Vibrationsmuster in der einen oder den mehreren bestäubungsbereiten Blumen zu induzieren, wobei das Vibrationsmuster durch Anlegen eines Luftstroms mit einem vorbestimmten Strömungsprofil induziert wird, das ein gerichteter und gezielter Luftstrom ist, der auf die eine oder die mehreren bestäubungsbereiten Blumen gerichtet ist, und Erzeugen von Betriebsdaten, die indikativ dafür sind; und Anlegen der Betriebsdaten an die eine oder die mehreren bestäubungsbereiten Blumen, um dadurch die eine oder die mehreren bestäubungsbereiten Blumen zu bestäuben; und
(ii) nach Bestimmen, dass die eine oder die mehreren Blüten bestäubt wurden, Hemmen einer Bestäubung anderer Blüten oder Verhindern eines Wachstums und Blühens anderer Blüten auf dem mindestens einen Abschnitt der Pflanze, indem ein Fluidstrom mit vorbestimmtem Temperatur-, Geschwindigkeits- und Raumprofil auf mindestens einen Teil des mindestens einen Abschnitts der Pflanze gerichtet wird.

## Revendications

1. Système de traitement de plantes (100), comprenant :
un appareil de traitement de plantes (102) comprenant :
un ou plusieurs dispositifs de traitement de plantes (103) configurés et fonctionnels pour appliquer un traitement à au moins une partie de la plante, dans lequel un ou plusieurs dispositifs desdits un ou plusieurs dispositifs de traitement de plantes sont configurés et fonctionnels pour appliquer le traitement en induisant de manière contrôlable un modèle de vibration dans l'au moins une partie de la plante, ledit modèle de vibration étant adapté à un type souhaité du traitement à appliquer ; et
un système de détection (104) comprenant un ou plusieurs capteurs (105) configurés et fonctionnels pour fournir des signaux de détection indiquant un état de ladite au moins une partie de la plante, lesdits un ou plusieurs capteurs comprenant un capteur optique (105a) configuré et fonctionnel pour fournir les signaux de détection indiquant des données d'image de ladite au moins une partie de la plante ; et
un système de commande configuré et fonctionnel pour une communication de données avec ledit appareil de traitement de plantes pour recevoir et traiter les signaux de détection produits par le système de détection, le traitement des signaux de détection comprenant la détermination d'un état de ladite au moins une partie de la plante, la définition de paramètres du modèle de vibration et le fonctionnement dudit au moins un dispositif de traitement de plantes pour induire le modèle de vibration correspondant au type de traitement souhaité à appliquer à ladite au moins une partie de la plante ;
le système de traitement de plantes étant **caractérisé en ce que**
ledit appareil de traitement de plantes comprend un ou plusieurs canaux
d'écoulement de fluide (106, 205) et un ou plusieurs applicateurs d'écoulement de
fluide (203), et
au moins un premier dispositif (103a) desdits un ou plusieurs dispositifs de traitement de plantes est configuré et fonctionnel pour induire le modèle de vibration en générant, par l'intermédiaire desdits un ou plusieurs canaux d'écoulement de fluide et un ou plusieurs applicateurs d'écoulement de fluide, un flux d'air ayant un profil d'écoulement prédéterminé, vers ladite au moins une partie de la plante, ledit profil d'écoulement prédéterminé du flux d'air étant un flux d'air directionnel et ciblé qui peut être dirigé vers une ou plusieurs régions spécifiques de ladite au moins une partie de la plante et induire le modèle de vibration dans ces régions spécifiques.

2. Système de traitement de plantes selon la revendication 1,
dans lequel au moins un second dispositif (103b) desdits un ou plusieurs dispositifs de traitement de plantes comprend un élément vibrant (302) configuré et fonctionnel pour entrer en contact avec ladite au moins une partie de la plante tout en vibrant afin d'induire ainsi de manière contrôlée ledit modèle de vibration dans ladite au moins une partie de la plante.

3. Système de traitement de plantes selon l'une quelconque des revendications précédentes, dans lequel ledit type de traitement souhaité est la pollinisation, dans lequel ledit premier ou second dispositif de traitement de plantes est configuré et fonctionnel en tant que dispositif de pollinisation de plantes, ledit système de commande est configuré et fonctionnel pour traiter les signaux de détection et, après avoir déterminé qu'une fleur dans ladite au moins une partie de la plante doit être pollinisée, générer des données opérationnelles correspondantes pour ledit premier ou second dispositif de traitement de plantes de sorte que ledit modèle de vibration induit est configuré pour provoquer la pollinisation d'au moins une fleur dans ladite au moins une partie de la plante.

4. Système de traitement de plantes selon l'une quelconque des revendications précédentes, dans lequel lesdits un ou plusieurs dispositifs de traitement de plantes comprennent un dispositif d'administration de substances (103c) configuré et fonctionnel pour délivrer ou pulvériser localement au moins une substance de traitement ou du pollen sur une ou plusieurs régions de ladite au moins une partie de la plante, ladite substance de traitement comprenant au moins l'un de ce qui suit : un médicament pour traiter les maladies des plantes, une hormone végétale induisant la croissance des plantes, un pesticide qui tue les nuisibles, ou une substance nuisible aux plantes qui empêche la croissance et/ou la pollinisation.

5. Système de traitement de plantes selon la revendication 4, dans lequel ledit dispositif d'administration de substances utilise lesdits un ou plusieurs canaux d'écoulement de fluide et lesdits un ou plusieurs applicateurs d'écoulement de fluide pour administrer ou pulvériser la substance de traitement ou le pollen.

6. Système de traitement de plantes selon l'une quelconque des revendications précédentes, dans lequel ledit système de commande est configuré et fonctionnel pour définir au moins l'un des paramètres suivants du modèle de vibration : la fréquence, l'amplitude et la durée du modèle de vibration.

7. Système de traitement de plantes selon l'une quelconque des revendications précédentes, dans lequel ledit système de commande est configuré et fonctionnel pour définir au moins l'un des paramètres suivants du modèle de vibration : nombre de trains d'impulsions d'air, intervalle de temps entre les trains d'impulsions, nombre d'impulsions dans chaque train d'impulsions, intervalle de temps entre deux impulsions dans chaque train d'impulsions, amplitude de pression dans chaque impulsion, durée de chaque impulsion.

8. Système de traitement de plantes selon l'une quelconque des revendications précédentes, dans lequel ladite au moins une partie de la plante étant traitée est située dans un champ de vision du capteur optique, ledit capteur optique et le canal d'écoulement de fluide sont configurés avec une orientation relative fixe prédéterminée comprenant au moins l'un d'un décalage ou d'une différence angulaire entre l'axe de visée du capteur optique et l'axe de propagation du flux d'air directionnel et ciblé.

9. Système de traitement de plantes selon la revendication 8, ayant au moins l'une des configurations suivantes :
a) un plan de collecte de lumière dudit capteur optique est situé de manière adjacente à une ouverture de sortie de fluide dudit applicateur d'écoulement de fluide, et
b) ledit capteur optique et une ouverture de sortie de fluide dudit applicateur d'écoulement de fluide sont attachés de manière fixe.

10. Système de traitement de plantes selon l'une quelconque des revendications précédentes, dans lequel lesdits un ou plusieurs dispositifs de traitement de plantes comprennent en outre un dispositif de transport de pollen (103d) configuré et fonctionnel pour collecter le pollen à partir d'un conteneur sur un véhicule ou dans une zone agricole et délivrer le pollen collecté à un pistil d'au moins une fleur dans ladite au moins une partie de la plante.

11. Système de traitement de plantes selon la revendication 10, dans lequel ledit dispositif de transport de pollen possède une surface à motifs configurée pour faire adhérer le pollen collecté à ladite surface.

12. Système de traitement de plantes selon l'une quelconque des revendications précédentes, dans lequel ledit système de détection comprend en outre un ou plusieurs capteurs environnementaux configurés et fonctionnels pour fournir les signaux de détection indiquant une ou plusieurs conditions environnementales à proximité de ladite au moins une partie de la plante.

13. Système de traitement de plantes selon la revendication 12, dans lequel lesdits un ou plusieurs dispositifs de traitement de plantes comprennent en outre un dispositif de conditionnement de l'environnement configuré et fonctionnel pour modifier au moins l'une de la température et de l'humidité d'un environnement de ladite au moins une partie de la plante.

14. Système de traitement de plantes selon la revendication 13, dans lequel ledit dispositif de conditionnement de l'environnement utilise lesdits un ou plusieurs canaux d'écoulement de fluide et lesdits un ou plusieurs applicateurs d'écoulement de fluide pour modifier la température ou l'humidité par écoulement d'air.

15. Système de traitement de plantes selon l'une quelconque des revendications 4 à 14, dans lequel ledit système de détection est **caractérisé par** au moins l'un de ce qui suit : a) le système de détection comprend un ou plusieurs capteurs environnementaux configurés et fonctionnels pour fournir les signaux de détection indiquant une ou plusieurs conditions environnementales à proximité de ladite au moins une partie de la plante, dans lequel lesdits signaux de détection indiquent des conditions défavorables à la pollinisation, et dans lequel ledit système de commande génère des données opérationnelles pour ledit dispositif d'administration de substances afin de délivrer ou de pulvériser une hormone qui induit une croissance de fruit parthénocarpique, et b) le système de détection fournit lesdits signaux de détection indiquant une maladie de ladite au moins une partie de la plante ou un nuisible dans un environnement de ou sur ladite au moins une partie de la plante, et dans lequel ledit système de commande génère des données opérationnelles pour ledit dispositif d'administration de substances afin de délivrer ou de pulvériser un médicament ou un pesticide respectivement.

16. Système de traitement de plantes selon l'une quelconque des revendications précédentes, dans lequel ledit appareil de traitement de plantes comprend en outre au moins un d'un ensemble de stérilisation, de nettoyage ou de désinfection (911) configuré et fonctionnel pour respectivement stériliser, nettoyer ou désinfecter lesdits un ou plusieurs dispositifs de traitement de plantes, l'ensemble comprenant au moins l'un de ce qui suit : une soufflerie d'air chaud (912), un applicateur de produit de nettoyage et un pulvérisateur de produit de nettoyage, de désinfection ou de stérilisation.

17. Système de traitement de plantes selon l'une quelconque des revendications précédentes, dans lequel ledit appareil de traitement de plantes comprend un ensemble de navigation et de suivi (1000) configuré et fonctionnel pour amener l'appareil de traitement des plantes à proximité de ladite au moins une partie de la plante afin de permettre ainsi le traitement de ladite au moins une partie de la plante par le système de traitement de plantes, le système de traitement de plantes étant **caractérisé par** au moins l'un de ce qui suit :
a) ledit ensemble de navigation et de suivi comprend un bras robotisé (901) portant ledit appareil de traitement de plantes, et dans lequel ledit système de commande est configuré et fonctionnel pour déplacer de manière contrôlée le bras robotisé en trois dimensions,
b) ledit ensemble de navigation et de suivi comprend un véhicule terrestre (1001) configuré et fonctionnel pour transporter de manière contrôlée l'appareil de traitement de plantes jusqu'à proximité de ladite au moins une partie de la plante,
c) ledit ensemble de navigation et de suivi comprend au moins l'un de ce qui suit : un ou plusieurs capteurs optiques (1014) et un capteur de positionnement,
d) ledit ensemble de navigation et de suivi comprend un bras robotisé portant ledit appareil de traitement de plantes et une unité à moment d'inertie configurée et fonctionnelle pour déterminer la trajectoire spatiale du bras robotisé afin d'optimiser ainsi le processus de traitement des plantes, le temps et l'énergie.

18. Système de traitement de plantes selon l'une quelconque des revendications précédentes, dans lequel ledit système de commande est **caractérisé par** au moins l'un de ce qui suit :
a) le système de commande est configuré et fonctionnel pour déterminer, sur la base desdits signaux de détection, si au moins une fleur sur ladite partie de la plante est prête pour la pollinisation, en comparant lesdits signaux de détection avec des données de référence comprenant des images de fleurs prêtes pour la pollinisation, ou en traitant lesdites données d'image pour identifier la présence d'une fleur dans la ou les images et identifier la préparation de la fleur ou des fleurs pour la pollinisation en identifiant les paramètres de la fleur indiquant l'existence ou l'absence de pollinisation ou en utilisant l'intelligence artificielle entraînée, et
b) le système de commande est configuré et fonctionnel pour analyser les signaux de détection provenant d'au moins le capteur optique et déterminer un état de ladite au moins une partie de la plante pendant et après le traitement, et générer les données de retour correspondantes, ce qui permet de prendre des décisions concernant la modification d'au moins un paramètre du traitement affectant le modèle de vibration induit dans la au moins une partie de la plante.

19. Système de traitement de plantes selon l'une quelconque des revendications précédentes, dans lequel lesdits un ou plusieurs dispositifs de traitement de plantes comprennent en outre un dispositif d'inhibition de la pollinisation (103e) configuré et fonctionnel pour empêcher la pollinisation d'une ou plusieurs fleurs ou empêcher la croissance et la floraison de fleurs supplémentaires dans ladite au moins une partie de la plante, tout en minimisant les dommages aux parties voisines de la plante, le dispositif d'inhibition de la pollinisation étant **caractérisé par** au moins l'un de ce qui suit :
a) le dispositif d'inhibition de la pollinisation comprend un dispositif laser (702, 701) configuré et fonctionnel pour irradier ladite au moins une partie de la plante avec des paramètres laser prédéterminés afin d'endommager ainsi ladite au moins une partie de la plante,
b) le dispositif d'inhibition de la pollinisation est configuré et fonctionnel pour générer, par l'intermédiaire desdits un ou plusieurs canaux d'écoulement de fluide, un écoulement d'air ayant une température élevée prédéterminée tout en maintenant la directionnalité de l'écoulement d'air en contrôlant la taille de la sortie d'écoulement d'air, afin d'endommager une ou plusieurs régions de ladite au moins une partie de la plante et d'empêcher la pollinisation d'une ou de plusieurs fleurs ou d'empêcher la croissance et la floraison de fleurs supplémentaires dans ladite au moins une partie de la plante, tout en minimisant les dommages causés aux parties voisines de la plante.

20. Appareil de traitement de plantes comprenant :
un ou plusieurs dispositifs de traitement de plantes (103, 103e), configurés et fonctionnels pour appliquer un traitement à au moins une partie d'une plante ;
dans lequel un ou plusieurs dispositifs desdits un ou plusieurs dispositifs de traitement de plantes sont configurés et fonctionnels pour appliquer le traitement en induisant de manière contrôlable un modèle de vibration dans l'au moins une partie de la plante, ledit modèle de vibration étant adapté à un type souhaité du traitement à appliquer ;
un système de détection (104) comprenant un ou plusieurs capteurs (105) configurés et fonctionnels pour fournir des signaux de détection indiquant un état de ladite au moins une partie de la plante, lesdits un ou plusieurs capteurs comprenant un capteur optique (105a) configuré et fonctionnel pour fournir les signaux de détection indiquant des données d'image de ladite au moins une partie de la plante ; et
un utilitaire de communication pour la communication de données avec un système de commande afin de transmettre les signaux de détection au système de commande et de recevoir du système de commande des données opérationnelles pour ledit au moins un dispositif de traitement de plantes induisant le modèle de vibration correspondant au type souhaité du traitement à appliquer à ladite au moins une partie de la plante ;
l'appareil de traitement de plantes étant **caractérisé en ce que** :
ledit appareil de traitement de plantes comprend un ou plusieurs canaux d'écoulement de fluide (106, 205) et un ou plusieurs applicateurs d'écoulement de fluide (203), et
au moins un premier dispositif (103a) desdits un ou plusieurs dispositifs de traitement de plantes est configuré et fonctionnel pour induire le modèle de vibration en générant, par l'intermédiaire desdits un ou plusieurs canaux d'écoulement de fluide et un ou plusieurs applicateurs d'écoulement de fluide, un flux d'air ayant un profil d'écoulement prédéterminé, vers ladite au moins une partie de la plante, ledit profil d'écoulement prédéterminé du flux d'air étant un flux d'air directionnel et ciblé qui peut être dirigé vers une ou plusieurs régions spécifiques de ladite au moins une partie de la plante et induire le modèle de vibration dans ces régions spécifiques.

21. Appareil de traitement de plantes selon la revendication 20, dans lequel lesdits un ou plusieurs dispositifs de traitement de plantes (103, 103e) sont configurés et fonctionnels pour causer des dommages ciblés à au moins une partie de ladite au moins une partie de la plante en soufflant, via un ou plusieurs desdits canaux d'écoulement de fluide (106, 205) et un ou plusieurs applicateurs d'écoulement de fluide (203), de l'air à haute température et à profil d'écoulement prédéterminés sur l'au moins une partie de l'au moins une partie de la plante, lesdites données opérationnelles pour ledit au moins un dispositif de traitement de plantes étant configurées pour causer les dommages ciblés à ladite au moins une partie de l'au moins une partie de la plante sur la base d'une ou plusieurs conditions suivantes de ladite au moins une partie de la plante : maladie, nuisible, état des fleurs, nombre de fleurs, nombre de fleurs déjà pollinisées.

22. Appareil de traitement de plantes selon la revendication 20 ou 21, dans lequel lesdits un ou plusieurs dispositifs de traitement de plantes comprennent en outre au moins l'un de ce qui suit : un laser (702) aux paramètres d'intensité et/ou de longueur d'onde prédéterminés, et l'administration d'une substance.

23. Appareil de traitement de plantes selon l'une quelconque des revendications 20 à 22, dans lequel ledit appareil de traitement de plantes comprend en outre un ensemble de navigation et de suivi (1000) configuré et fonctionnel pour amener l'appareil de traitement de plantes à proximité de ladite au moins une partie de la plante afin de permettre ainsi le traitement de ladite au moins une partie de la plante par le dispositif de traitement de plantes, ledit ensemble de navigation et de suivi comprend un ou plusieurs de ce qui suit :
a) un bras robotisé (901) portant ledit appareil de traitement de plantes et configuré et fonctionnel pour se déplacer en trois dimensions,
b) un véhicule terrestre (1001) configuré et fonctionnel pour transporter de manière contrôlée l'appareil de traitement de plantes jusqu'à proximité de ladite au moins une partie de la plante,
c) au moins un de ce qui suit : un ou plusieurs capteurs optiques (1014) et un capteur de positionnement,
d) un bras robotisé portant ledit appareil de traitement de plantes et une unité à moment d'inertie configurée et fonctionnelle pour déterminer la trajectoire spatiale du bras robotisé afin d'optimiser ainsi le processus de traitement des plantes, le temps et l'énergie.

24. Appareil de traitement de plantes selon l'une quelconque des revendications 20 à 23, dans lequel ladite au moins une partie de l'au moins une partie de la plante est une fleur unique ou une région d'une fleur unique.

25. Procédé de traitement de plantes comprenant :
- l'acquisition de données de détection comprenant des données d'image d'au moins une partie d'une plante ;
- l'analyse desdites données de détection pour déterminer si une ou plusieurs fleurs sur ladite au moins une partie de la plante satisfont à l'une des conditions suivantes : (i) lesdites une ou plusieurs fleurs sont prêtes à être pollinisées, ou (ii) lesdites une ou plusieurs fleurs ont été pollinisées ;
- l'exécution sélective de l'une de ce qui suit :
(i) lors de la détection d'une ou de plusieurs fleurs prêtes à être pollinisées, la définition de paramètres de vibration destinés à induire un modèle de vibration souhaité dans lesdites une ou plusieurs fleurs prêtes à être pollinisées, le modèle de vibration étant induit par l'application d'un flux d'air ayant un profil d'écoulement prédéterminé, à savoir un flux d'air directionnel et ciblé dirigé vers les une ou plusieurs fleurs prêtes à être pollinisées, et la génération de données opérationnelles indicatives de celle-ci ; et l'application desdites données opérationnelles auxdites une ou plusieurs fleurs prêtes à être pollinisées afin de polliniser ainsi lesdites une ou plusieurs fleurs prêtes à être pollinisées ; et
(ii) après avoir déterminé que les une ou plusieurs fleurs ont été pollinisées, l'inhibition de la pollinisation d'une autre ou d'autres fleurs ou l'empêchement de la croissance et de la floraison d'une autre ou d'autres fleurs sur ladite au moins une partie de la plante, en dirigeant un écoulement de fluide de profil de température, de vitesse et de profil spatial prédéterminés vers au moins une partie de ladite au moins une partie de la plante.
